# EUROPEAN PATENT SPECIFICATION

(11) **EP 2 324 016 B1**
(45) Date of publication and mention of the grant of the patent: **25.04.2012**
(21) Application number: 09772404.1
(22) Date of filing: 29.06.2009
(51) Int. Cl.: C07D 413/10, C07D 413/14, A61K 31/443, A61K 31/381, A61K 31/422, A61P 31/04

(54) **3-(N-HETEROCYCLYL)-PYRROLIDINYL-PHENYL-OXAZOLIDINONES AS ANTIBACTERIAL AGENTS**
3-(N-HETEROCYCLYL)PYRROLIDINYLPHENYLOXAZOLIDINONE ALS ANTIBAKTERIELLE MITTEL
3-(N-HÉTÉROCYCLYL)-PYRROLIDINYL-PHÉNYL-OXAZOLIDINONES COMME AGENTS ANTIBACTÉRIENS

(30) Priority: 01.07.2008 EP 08159447
(43) Date of publication of application: 25.05.2011
(73) Proprietor: Ferrer Internacional, S.A., 08028 Barcelona (ES)
(72) Inventor: CANO, Montserrat, E-08691 Monistrol (Barcelona) (ES); PALOMER, Albert, E-08014 Barcelona (ES); GUGLIETTA, Antonio, E-08750 Molins de Rei (Barcelona) (ES)
(74) Representative: ZBM Patents
(86) International application number: PCT/EP2009/058126
(87) International publication number: WO 2010/000704

(56) References cited:
- WO-A-96/13502
- US-A- 5 688 792
- DU YU ET AL: "Synthesis and antibacterial activity of linezolid analogUES" BIOORGANIC & MEDICINAL CHEMISTRY LETTERS, PERGAMON, ELSEVIER SCIENCE, GB, vol. 12, 1 January 2002 (2002-01-01), pages 857-859, XP002245432 ISSN: 0960-894X

## Description

### TECHNICAL FIELD

This invention is directed to antimicrobial oxazolidinone compounds which are active against Gram-positive and some Gram-negative bacteria, showing specifically a potent activity against linezolid-resistant (LNZ-R) strains of Gram-positive bacteria and more specifically against Gram-positive pathogenic respiratory bacteria.

### BACKGROUND ART

Oxazolidinones are Gram-positive antimicrobial agents. Oxazolidinones bind to the 50S subunit of the prokaryotic ribosome, preventing formation of the initiation complex for protein synthesis. This is a novel mode of action. Other protein synthesis inhibitors either block polypeptide extension or cause misreading of mRNA. Linezolid (N-[[(5S)-3-[3-fluoro-4-(4-morpholinyl)phenyl]-2-oxo-5-oxazolidinyl]methyl]acetamide), US5688792, is the first approved antimicrobial oxazolidinone for clinical use in the United States and elsewhere. The structural formula of linezolid is:

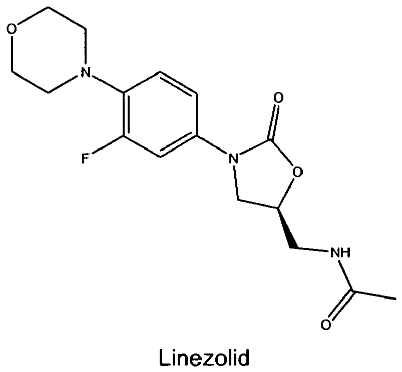

Linezolid minimal inhibitory concentrations (MICs) vary slightly with the test mode, laboratory, and significance attributed to thin hazes of bacterial survival, but all workers find that the susceptibility distributions are narrow and unimodal with MIC values between 0.5 and 4 µg/mL for *streptococci, enterococci* and *staphylococci.* Full activity is retained against Gram-positive *cocci* resistant to other antibiotics, including methicillin-resistant *staphylococci* and vancomycin-resistant *enterococci.* MICs are 2-8 µg/mL for *Moraxella, Pasteurella* and *Bacteroides* spp. but other Gram-negative bacteria are resistant as a result of endogenous efflux activity as well as the intake presented by Gram-negative bacteria outer membrane cell. Linezolid is indicated for the treatment of adult patients with the following infections: vancomycin-resistant *Enterococcus faecium* infections, including concurrent bacteremia; nosocomial pneumonia; complicated skin and skin structure infections; community-acquired pneumonia, including concurrent bacteremia; diabetic foot infections; and uncomplicated skin and skin structure infections.

Unfortunately, some Gram-positive bacteria such as *Staphylococcus aureus* (LNZ-R 432), *Haemophylus influenzae* (ATCC 49247), *Bacteroides fragilis* (ATCC 25285), *Moraxella catarrhalis* (HCl-78), and *Enterococcus faecium* (LNZ-R) show an important resistance to linezolid, thus suggesting the need of new oxazolidinone compounds active in these strains. Some of them are the origin of severe and sometimes fatal infections such as sepsis and septic shock. Further, there is an increasing need for improved agents against Gram-positive pathogenic respiratory bacteria, like *Streptococcus pneumoniae, Haemophylus influenzae,* and *Moraxella catarrhalis.*

### SUMMARY OF THE INVENTION

Surprisingly the compounds of the present application are potent active antimicrobial agents showing a relevant activity against LNZ-R Gram-positive bacteria and more specifically against Gram-positive pathogenic respiratory bacteria. Differential characteristic properties of the compounds of the present invention versus linezolid indicate the potential use thereof in severe infections that cannot be properly treated with linezolid.

In a first aspect the present invention refers to a compound of formula (I), 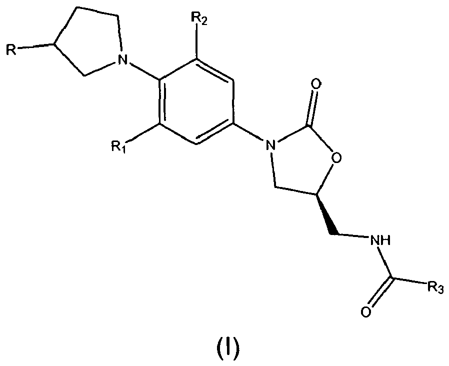 in free or pharmaceutically acceptable salt, solvate, hydrate, or enantiomeric form, wherein:
R is a N-linked 5-membered fully or partially unsaturated heterocyclic ring, containing 0 to 3 further nitrogen heteroatoms,
   which ring is optionally substituted on any available carbon atom with a substituent selected from linear or branched (1-6C)alkyl, (3-6C)cycloalkyl, halogen, OR₄, S(O)ₘR₅, COOH, COOR₆, COR₇, CONH₂, CONHR₈, CONR₉R₁₀, SO₂NH₂, SO₂NHR₁₁, SO₂NR₁₂R₁₃, NH₂, NHR₁₄, NR₁₅R₁₆, NHCOR₁₇, N(R₁₈)COR₁₉, NHSO₂R₂₀, N(R₂₁)SO₂R₂₂, CN, CF₃, NO₂, phenyl optionally substituted with up to three substituents independently selected from linear or branched (1-6C)alkyl, (3-6C)cycloalkyl, halogen, OR₂₃, S(O)ₙR₂₄, NH₂, NHR₂₅, NR₂₆R₂₇, NHCOR₂₈, N(R₂₉)COR₃₀, NHSO₂R₃₁, N(R₃₂)SO₂R₃₃, CN, CF₃, NO₂, and 5-6 membered heteroaryl group containing one to three heteroatoms selected from nitrogen, oxygen and sulfur, optionally substituted with up to three substituents independently selected from linear or branched (1-6C)alkyl, (3-6C)cycloalkyl, halogen, OR₃₄, S(O)ₚR₃₅, NH₂, NHR₃₆, NR₃₇R₃₈, NHCOR₃₉, N(R₄₀)COR₄₁, NHSO₂R₄₂, N(R₄₃)SO₂R₄₄, CN, CF₃, and NO₂,
   said ring being optionally fused with a phenyl or 5-6 membered fully or partially unsaturated heterocycle containing one to three heteroatoms selected from nitrogen, oxygen and sulfur to form a benzo-fused or hetero-fused system, wherein the benzo- or hetero- moiety is optionally substituted with up to three substituents independently selected from linear or branched (1-6C)alkyl, (3-6C)cycloalkyl, halogen, OR₄₅, S(O)_{q}R₄₆, NH₂, NHR₄₇, NR₄₈R₄₉, NHCOR₅₀, N(R₅₁)COR₅₂, NHSO₂R₅₃, N(R₅₄)SO₂R₅₅, CN, CF₃, and NO₂;
   R₁ and R₂ are radicals identical or different and are independently selected from hydrogen, and fluorine;
   R₃ is a linear or branched (1-6C)alkyl group optionally substituted by a group selected from fluorine, hydroxy, and OR₅₆;
   R₄ to R₅₆ are identical or different linear or branched (1-6C)alkyl groups; or R₉+R₁₀, R₁₂+R₁₃, R₁₅+R₁₆, R₂₆+R₂₇, R₃₇+R₃₈, and R₄₈+R₄₉ together with the nitrogen atom carrying them, form a monocyclic 5-, 6- or 7-membered saturated heterocycle optionally containing in the cyclic system a second hetero atom selected from oxygen and nitrogen; and
   m, n, p and q are identical or different integers independently selected from 0, 1,and2.

In a second aspect the present invention refers to a process for preparing a compound of formula (I) in free or pharmaceutically acceptable salt, solvate, hydrate, or enantiomeric form that comprises:
(i) a) reacting an intermediate of formula (II), 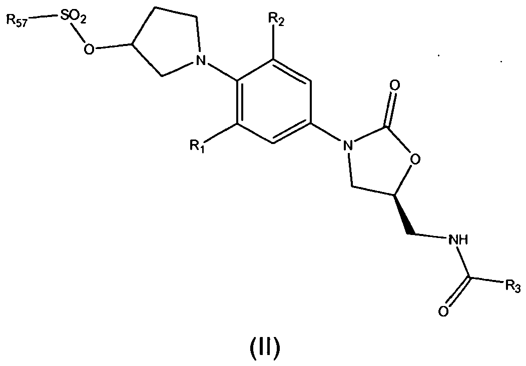 wherein R₁, R₂ and R₃ are as defined above, and R₅₇ is selected from methyl, phenyl, p-tolyl, p-bromophenyl, p-nitrophenyl, trifluoromethyl, and 2,2,2-trifluoroethyl, with an intermediate of formula RH (III), wherein R is as defined above; or
   b) reacting an intermediate of formula (IV), 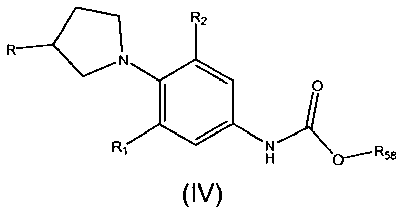 wherein R, R₁ and R₂ are as defined above and R₅₈ is selected from linear or branched (1-6C)alkyl, and benzyl optionally substituted in the phenyl ring by up to three linear or branched (1-6C)alkyl groups, with an intermediate of formula (V), 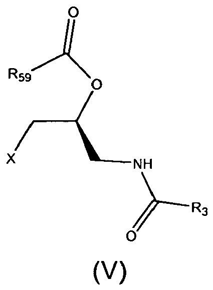 wherein R₃ is as defined above, R₅₉ is a linear or branched (1-6C)alkyl group, and X is a halogen atom; and
(ii) recovering the resultant compound of formula (I) in free or pharmaceutically acceptable salt, solvate, hydrate, or enantiomeric form.

In a third aspect the present invention refers to a pharmaceutical composition comprising a therapeutically effective amount of the compound of general formula (I) according to the first aspect of the invention, together with the appropriate amounts of pharmaceutical excipients or carriers.

In a fourth aspect the present invention refers to a compound of formula (I) according to the first aspect of the invention, for use as a medicament.

In an fifth aspect the present invention refers to the use of a compound of formula (I) according to the first aspect of the invention for the manufacture of a medicament for the treatment of bacterial infections in an animal or human. This aspect may also be formulated as a compound of formula (I) according to the first aspect of the invention for use in the treatment of bacterial infections.

### DETAILED DESCRIPTION OF THE INVENTION

The term "pharmaceutically acceptable salts" used herein encompasses any salt formed from organic and inorganic acids, such as hydrobromic, hydrochloric, phosphoric, nitric, sulfuric, acetic, adipic, aspartic, benzenesulfonic, benzoic, citric, ethanesulfonic, formic, fumaric, glutamic, lactic, maleic, malic, malonic, mandelic, methanesulfonic, 1,5-naphthalendisulfonic, oxalic, pivalic, propionic, p-toluenesulfonic, succinic, tartaric acids, and the like, and any salt formed from organic and inorganic bases, such as the alkali metal and alkaline earth metal salts, especially the sodium and potassium salts, ammonium salts and salts of amines, including lower alkylated amines, such as methylamine, ethylamine, trimethylamine and the like, hydroxyloweralkylamines, such as ethanolamine and diethanolamine, and heterocyclic amines, such as morpholine and piperazine.

In a preferred embodiment, the present invention refers to a compound according to the first aspect of the invention wherein R is selected from benzotriazolyl, 1-imidazolyl, 4-acetylpyrazol-1-yl, 4-bromopyrazol-1-yl, 4-nitropyrazolyl, 3-trifluoromethyl-pyrazol-1-yl, 3-phenylpyrazol-1-yl, 3-(2-fluorophenyl)-pyrazol-1-yl, 3-(4-trifluoromethyl-phenyl)-pyrazol-1-yl, 4-(4-fluorophenyl)-pyrazol-1-yl, 4-(2-methoxyphenyl)-pyrazol-1-yl, 4-(4-nitrophenyl)-pyrazol-1-yl, 4-(2-trifluoromethyl-phenyl)-pyrazol-1-yl, 4-pyrazin-2-yl-pyrazol-1-yl, 4-pyridin-4-yl-pyrazol-1-yl, 4-pyrimidin-4-yl-pyrazol-1-yl, 1-tetrazolyl, 2-tetrazolyl, 5-methyltetrazol-2-yl, 5-methylsulfanylltetrazol-2-yl, 5-phenyltetrazol-2-yl, 5-p-tolyltetrazol-2-yl, 5-thiophen-2-yl-tetrazol-2-yl, 1-triazolyl, 2-triazolyl, [1,2,3]triazol-1-yl, [1,2,3]triazol-2-yl, (3-cyanophenyl)-[1,2,3]triazol-2-yl], 4-pyridin-2-yl-[1,2,3]triazol-2-yl, and [1,2,4]triazol-1-yl is R₁ is fluorine, R₂ is selected from fluorine and hydrogen, and R₃ is methyl. Preferably, the compound according to the first aspect of the invention is selected from the group consisting of:
*N*-((5S)-3-{3-fluoro-4-[3-(2-triazolyl)pyrrolidin-1-yl]-phenyl}-2-oxo-5-oxazolidinyl methyl) acetamide;
*N*-((5S)-3-{3-fluoro-4-[3-(1-triazolyl)pyrrolidin-1-yl]-phenyl}-2-oxo-5-oxazolidinyl methyl) acetamide;
*N*-((5S)-3-[3-fluoro-4-[3-(1-imidazolyl)pyrrolidin-1-yl]-phenyl}-2-oxo-5-oxazolidinyl methyl) acetamide;
*N*-((5S)-3-{3-fluoro-4-[3-(2-tetrazolyl)pyrrolidinyl]-phenyl}-2-oxo-5-oxazolidinyl methyl) acetamide;
*N*-((5*S*)-3-(3-fluoro-4-[3-(1-tetrazolyl)pyrrolidinyl]-phenyl}-2-oxo-5-oxazolidinyl methyl) acetamide;
*N*-{(5*S*)-3-[3-fluoro-4-(3-[1,2, 4]triazol-1-yl-pyrrolidin-1-yl)-phenyl]-2-oxo-5-oxazolidinylmethyl} acetamide;
*N*-((5*S*)-3-{3-fluoro-4-[3-(benzotriazolyl)pyrrolidin-1-yl]-phenyl}-2-oxo-5-oxazolidinyl methyl) acetamide;
*N*-((5*S*)-3-{3-fluoro-4-[3-(4-nitropyrazolyl)pyrrolidinyl]-phenyl}-2-oxo-5-oxazolidinyl methyl) acetamide ;
*N*-((5*S*)-3-{3-fluoro-4-[3-(5-p-tolyltetrazol-2-yl)pyrrolidin-1-yl]-phenyl}-2-oxo-5-oxazolidinylmethyl) acetamide ;
*N*-((55)-3-(3-fluoro-4-[3-(4-pyrimidin-4-yl-pyrazol-1-yl)pyrrolidin-1-yl]-phenyl}-2-oxo-5-oxazolidinylmethyl) acetamide;
*N*-((5S)-3-{3-fluoro-4-[3-(4-pyrazin-2-yl-pyrazol-1-yl)pyrrolidin-1-yl]-phenyl}-2-oxo-5-oxazolidinylmethyl) acetamide;
*N*-((5S)-3-{3-fluoro-4-[3-(5-phenyltetrazol-2-yl)pyrrolidin-1-yl]-phenyl}-2-oxo-5-oxazolidinylmethyl) acetamide;
*N*-((5S)-3-{3-fluoro-4-[3-(5-methylsulfanylltetrazol-2-yl)pyrrolidin-1-yl]-phenyl}-2-oxo-5-oxazolidinylmethyl) acetamide;
*N*-((5S)-3-{3-fluoro-4-[3-(5-thiophen-2-yl-tetrazol-2-yl)pyrrolidin-1-yl]-phenyl}-2-oxo-5-oxazolidinylmethyl) acetamide;
*N*-((5S)-3-{3-fluoro-4-[3-(5-methyltetrazol-2-yl)pyrrolidin-1-yl]-phenyl}-2-oxo-5-oxazolidinylmethyl) acetamide;
*N*-((5*S*)-3-{3-fluoro-4-[3-(4-bromopyrazol-1-yl)pyrrolidin-1-yl]-phenyl}-2-oxo-5-oxazolidinylmethyl) acetamide;
*N*-((5*S*)-3-{3-fluoro-4-[3-(4-pyridin-4-yl-pyrazol-1-yl)pyrrolidin-1-yl]-phenyl)-2-oxo-5-oxazolidinylmethyl) acetamide;
N-[(5*S*)-3-(3-fluoro-4-{3-[4-(4-nitrophenyl)-pyrazol-1-yl]pyrrolidin-1-yl}-phenyl)-2-oxo-5-oxazolidinylmethyl] acetamide;
*N*-[(5S)-3-(3-fluoro-4-{3-[4-(2-trifluoromethyl-phenyl)-pyrazol-1-yl]pyrrolidin-1-yl}-phenyl)-2-oxo-5-oxazolidinylmethyl] acetamide;
*N*-[(5S)-3-(3-fluoro-4-{3-[4-(2-methoxyphenyl)-pyrazol-1-yl]pyrrolidin-1-yl)-phenyl)-2-oxo-5-oxazolidinylmethyl] acetamide;
*N*-((5S)-3-{3-fluoro-4-[3-(4-acetylpyrazol-1-yl)pyrrolidin-1-yl]-phenyl}-2-oxo-5-oxazolidinylmethyl) acetamide;
*N*-((5S)-3-{3-fluoro-4-[3-(3-phenylpyrazol-1-yl)pyrrolidin-1-yl]-phenyl}-2-oxo-5-oxazolidinylmethyl) acetamide;
*N*-[(5S)-3-(3-fluoro-4-{3-[4-(4-fluorophenyl)-pyrazol-1-yl]pyrrolidin-1-yl}-phenyl)-2-oxo-5-oxazolidinylmethyl] acetamide;
*N*-[(5S)-3-(3-fluoro-4-{3-[3-(2-fluorophenyl)-pyrazol-1-yl]pyrrolidin-1-yl}-phenyl)-2-oxo-5-oxazolidinylmethyl] acetamide;
*N*-((5S)-3-{3-fluoro-4-[3-(3-trifluoromethyl-pyrazol-1-yl)pyrrolidin-1-yl]-phenyl}-2-oxo-5-oxazolidinylmethyl) acetamide;
*N*-[(5S)-3-(3-fluoro-4-{3-[3-(4-trifluoromethyl-phenyl)-pyrazol-1-yl]pyrrolidin-1-yl}-phenyl)-2-oxo-5-oxazolidinylmethyl] acetamide;
*N*-((5*S*)-3-{3-fluoro-4-[3-(4-pyridin-2-yl-[1,2,3]triazol-2-yl)pyrrolidin-1-yl]-phenyl}-2-oxo-5-oxazolidinylmethyl) acetamide;
*N*-[(5S)-3-(3-fluoro-4-{3-[4-(3-cyanophenyl)-[1,2,3]triazol-2-yl]pyrrolidin-1-yl}-phenyl)-2-oxo-5-oxazolidinylmethyl] acetamide;
*N*-{(5S)-3-[3, 5-difluoro-4-[3-([1,2,3]triazol-2-yl pyrrolidin-1-yl) phenyl]-2-oxo-5-oxazolidinyl methyl} acetamide;
*N*-{(5S)-3-[3, 5-difluoro-4-[3-([1,2,3]triazol-1-yl pyrrolidin-1-yl) phenyl]-2-oxo-5-oxazolidinyl methyl} acetamide;
*N*-{(5S)-3-[3, 5-difluoro-4-[3-(tetrazol-2-yl pyrrolidin-1-yl) phenyl]-2-oxo-5-oxazolidinyl methyl} acetamide;
*N*-{(5S)-3-[3, 5-difluoro-4-[3-(tetrazol-1-yl pyrrolidin-1-yl) phenyl]-2-oxo-5-oxazolidinyl methyl} acetamide ;
*N*-((5S)-3-{3-fluoro-4-[3(R)-(2-triazolyl)pyrrolidin-1-yl]-phenyl}-2-oxo-5-oxazolidinyl methyl) acetamide;
*N*-((5S)-3-{3-fluoro-4-[3(R)-(1-triazolyl)pyrrolid in-1-yl]-phenyl}-2-oxo-5-oxazolidinyl methyl) acetamide;
*N*-((5S)-3-[3-fluoro-4-[3(R)-(1-imidazolyl)pyrrolidin-1-yl]-phenyl}-2-oxo-5-oxazolidinyl methyl) acetamide;
*N*-((5S)-3-{3-fluoro-4-[3(R)-(2-tetrazolyl)pyrrolidinyl]-phenyl}-2-oxo-5-oxazolidinyl methyl) acetamide;
*N*-((5S)-3-{3-fluoro-4-[3(R)-(1-tetrazolyl)pyrrolidinyl]-phenyl}-2-oxo-5-oxazolidinyl methyl) acetamide;
*N*-{(5S)-3-[3-fluoro-4-(3(R)-[1, 2, 4]triazol-1-yl-pyrrolidin-1-yl)-phenyl]-2-oxo-5-oxazolidinylmethyl} acetamide;
*N*-((5S)-3-{3-fluoro-4-[3(R)-(benzotriazolyl)pyrrolidin-1-yl]-phenyl}-2-oxo-5-oxazolidinyl methyl) acetamide;
*N*-((5S)-3-{3-fluoro-4-[3(R)-(4-nitropyrazolyl)pyrrolidinyl]-phenyl}-2-oxo-5-oxazolidinyl methyl) acetamide ;
*N*-((5S)-3-{3-fluoro-4-[3(R)-(5-p-tolyltetrazol-2-yl)pyrrolidin-1-yl]-phenyl}-2-oxo-5-oxazolidinylmethyl) acetamide;
*N*-((5S)-3-{3-fluoro-4-[3(R)-(4-pyrimidin-4-yl-pyrazol-1-yl)pyrrolidin-1-yl]-phenyl}-2-oxo-5-oxazolidinylmethyl) acetamide;
*N*-((5S)-3-{3-fluoro-4-[3(R)-(4-pyrazin-2-yl-pyrazol-1-yl)pyrrolidin-1-yl]-phenyl}-2-oxo-5-oxazolidinylmethyl) acetamide;
*N*-((5S)-3-{3-fluoro-4-[3(R)-(5-phenyltetrazol-2-yl)pyrrolidin-1-yl]-phenyl}-2-oxo-5-oxazolidinylmethyl) acetamide;
*N*-((5S)-3-{3-fluoro-4-[3(R)-(5-methylsulfanylltetrazol-2-yl)pyrrolidin-1-yl]-phenyl}-2-oxo-5-oxazolidinylmethyl) acetamide;
*N*-((5S)-3-(3-fluoro-4-[3(R)-(5-thiophen-2-yl-tetrazol-2-yl)pyrrolidin-1-yl]-phenyl}-2-oxo-5-oxazolidinylmethyl) acetamide;
*N*-((5S)-3-(3-fluoro-4-[3(R)-(5-methyltetrazol-2-yl)pyrrolidin-1-yl]-phenyl}-2-oxo-5-oxazolidinylmethyl) acetamide;
*N*-((5S)-3-{3-fluoro-4-[3(R)-(4-bromopyrazol-1-yl)pyrrolidin-1-yl]-phenyl}-2-oxo-5-oxazolidinylmethyl) acetamide;
*N*-((5S)-3-{3-fluoro-4-[3(R)-(4-pyridin-4-yl-pyrazol-1-yl)pyrrolidin-1-yl]-phenyl}-2-oxo-5-oxazolidinylmethyl) acetamide;
*N*-[(5S)-3-(3-fluoro-4-{3(R)-[4-(4-nitrophenyl)-pyrazol-1-yl]pyrrolidin-1-yl}-phenyl)-2-oxo-5-oxazolidinylmethyl] acetamide;
*N*-[(5S)-3-(3-fluoro-4-{3(R)-[4-(2-trifluoromethyl-phenyl)-pyrazol-1-yl]pyrrolidin-1-yl}-phenyl)-2-oxo-5-oxazolidinylmethyl] acetamide;
*N*-[(5S)-3-(3-fluoro-4-{3(R)-[4-(2-methoxyphenyl)-pyrazol-1-yl]pyrrolidin-1-yl}-phenyl)-2-oxo-5-oxazolidinylmethyl] acetamide;
*N*-((5S)-3-{3-fluoro-4-[3(R)-(4-acetylpyrazol-1-yl)pyrrolidin-1-yl]-phenyl}-2-oxo-5-oxazolidinylmethyl) acetamide;
*N*-((5S)-3-{3-fluoro-4-[3(R)-(3-phenylpyrazol-1-yl)pyrrolidin-1-yl]-phenyl}-2-oxo-5-oxazolidinylmethyl) acetamide;
*N*-[(5S)-3-(3-fluoro-4-{3(R)-[4-(4-fluorophenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*R*)-[3-(2-fluorophenyl)-pyrazol-1-yl]pyrrolidin-1-yl}-phenyl)-2-oxo-5-oxazolidinylmethyl] acetamide;
*N*-((5*S*)-3-{3-fluoro-4-[3(*R*)-(3-trifluoromethyl-pyrazol-1-yl)pyrrolidin-1-yl]-phenyl}-2-oxo-5-oxazolidinylmethyl) acetamide;
*N*-[(5*S*)-3-(3-fluoro-4-{3(*R*)-[3-(4-trifluoromethyl-phenyl)-pyrazol-1-yl]pyrrolidin-1-yl}-phenyl)-2-oxo-5-oxazolidinylmethyl] acetamide;
*N*-((5*S*)-3-{3-fluoro-4-[3(*R*)-(4-pyridin-2-yl-[1,2,3]triazol-2-yl)pyrrolidin-1-yl]-phenyl}-2-oxo-5-oxazolidinylmethyl) acetamide;
*N*-[(5*S*)-3-(3-fluoro-4-{3(*R*)-[4-(3-cyanophenyl)-[1,2,3]triazol-2-yl]pyrrolidin-1-yl}-phenyl)-2-oxo-5-oxazolidinylmethyl] acetamide;
*N*-{(5*S*)-3-[3, 5-difluoro-4-[3(*R*)-([1,2,3]triazol-2-yl pyrrolidin-1-yl) phenyl]-2-oxo-5-oxazolidinyl methyl} acetamide;
*N*-{(5*S*)-3-[3, 5-difluoro-4-[3(R)-([1,2,3]triazol-1-yl pyrrolidin-1-yl) phenyl]-2-oxo-5-oxazolidinyl methyl} acetamide;
*N*-1(5*S*)-3-[3, 5-difluoro-4-[3(R)-(tetrazol-2-yl pyrrolidin-1-yl) phenyl]-2-oxo-5-oxazolidinyl methyl} acetamide;
*N*-{(5*S*)-3-[3, 5-difluoro-4-[3(*R*)-(tetrazol-1-yl pyrrolidin-1-yl) phenyl]-2-oxo-5-oxazolidinyl methyl} acetamide ;
*N*-((5*S*)-3-{3-fluoro-4-(3(*S*)-(2-triazolyl)pyrrolidin-1-yl]-phenyl}-2-oxo-5-oxazolidinyl methyl) acetamide;
*N*-((5*S*)-3-{3-fluoro-4-[3(S)-(1-triazolyl)pyrrolidin-1-yl]-phenyl}-2-oxo-5-oxazolidinyl methyl) acetamide;
*N*-((5*S*)-3-[3-fluoro-4-[3(S)-(1-imidazolyl)pyrrolidin-1-yl]-phenyl}-2-oxo-5-oxazolidinyl methyl) acetamide;
*N*-((5*S*)-3-{3-fluoro-4-[3(S)-(2-tetrazolyl)pyrrolidinyl]-phenyl}-2-oxo-5-oxazolidinyl methyl) acetamide;
*N*-((5*S*)-3-{3-fluoro-4-[3(S)-(1-tetrazolyl)pyrrolidinyl]-phenyl}-2-oxo-5-oxazolidinyl methyl) acetamide;
N-{(5S)-3-[3-fluoro-4-(3(*S*)-[1, 2, 4]triazol-1-yl-pyrrolidin-1-yl)-phenyl]-2-oxo-5-oxazolidinylmethyl} acetamide;
N-((5S)-3-{3-fluoro-4-[3(*S*)-(benzotriazolyl)pyrrolidin-1-yl]-phenyl}-2-oxo-5-oxazolidinyl methyl) acetamide;
N-((5S)-3-{3-fluoro-4-[3(*S*)-(4-nitropyrazolyl)pyrrolidinyl]-phenyl}-2-oxo-5-oxazolidinyl methyl) acetamide ;
N-((5S)-3-{3-fluoro-4-[3(*S*)-(5-p-tolyltetrazol-2-yl)pyrrolidin-1-yl]-phenyl}-2-oxo-5-oxazolidinylmethyl) acetamide ;
N-((5S)-3-{3-fluoro-4-[3(*S*)-(4-pyrimidin-4-yl-pyrazol-1-yl)pyrrolidin-1-yl]-phenyl}-2-oxo-5-oxazolidinylmethyl) acetamide;
*N*-((5*S*)-3-{3-fluoro-4-[3(S)-(4-pyrazin-2-yl-pyrazol-1-yl)pyrrolidin-1-yl]-phenyl}-2-oxo-5-oxazolidinylmethyl) acetamide;
*N*-((5*S*)-3-{3-fluoro-4-[3(S)-(5-phenyltetrazol-2-yl)pyrrolidin-1-yl]-phenyl}-2-oxo-5-oxazolidinylmethyl) acetamide;
N-((5*S*)-3-{3-fluoro-4-[3(*S*)-(5-methylsulfanylltetrazol-2-yl)pyrrolidin-1-yl]-phenyl}-2-oxo-5-oxazolidinylmethyl) acetamide;
*N*-((5*S*)-3-{3-fluoro-4-[3(*S*)-(5-thiophen-2-yl-tetrazol-2-yl)pyrrolidin-1-yl]-phenyl}-2-oxo-5-oxazolidinylmethyl) acetamide;
*N*-((5*S*)-3-{3-fluoro-4-[3(*S*)-(5-methyltetrazol-2-yl)pyrrolidin-1-yl]-phenyl}-2-oxo-5-oxazolidinylmethyl) acetamide;
*N*-((5*S*)-3-{3-fluoro-4-[3(*S*)-(4-bromopyrazol-1-yl)pyrrol idin-1-yl]-phenyl}-2-oxo-5-oxazolidinylmethyl) acetamide;
*N*-((5*S*)-3-{3-fluoro-4-[3(*S*)-(4-pyrid in-4-yl-pyrazol-1-yl)pyrrolidin-1-yl]-phenyl}-2-oxo-5-oxazolidinylmethyl) acetamide;
*N*-[(5*S*)-3-(3-fluoro-4-{3(*S*)-[4-(4-nitrophenyl)-pyrazol-1-yl]pyrrolidin-1-yl}-phenyl)-2-oxo-5-oxazolidinylmethyl] acetamide;
*N*-[(5S)-3-(3-fluoro-4-{3(*S*)-[4-(2-trifluoromethyl-phenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*S*)-[4-(2-methoxyphenyl)-pyrazol-1-yl]pyrrolidin-1-yl}-phenyl)-2-oxo-5-oxazolidinylmethyl] acetamide;
*N*-((5*S*)-3-{3-fluoro-4-[3(*S*)-(4-acetylpyrazol-1-yl)pyrrolidin-1-yl]-phenyl}-2-oxo-5-oxazolidinylmethyl) acetamide;
*N*-((5S)-3-{3-fluoro-4-[3(*S*)-(3-phenylpyrazol-1-yl)pyrrolidin-1-yl]-phenyl}-2-oxo-5-oxazolidinylmethyl) acetamide;
*N*-[(5*S*)-3-(3-fluoro-4-{3(*S*)-[4-(4-fluorophenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*S*)-[3-(2-fluorophenyl)-pyrazol-1-yl]pyrrolidin-1-yl}-phenyl)-2-oxo-5-oxazolidinylmethyl] acetamide;
*N*-((5*S*)-3-{3-fluoro-4-[3(S)-(3-trifluoromethyl-pyrazol-1-yl)pyrrolidin-1-yl]-phenyl}-2-oxo-5-oxazolidinylmethyl) acetamide;
*N*-((5*S*)-3-(3-fluoro-4-{3(S)-[3-(4-trifluoromethyl-phenyl)-pyrazol-1-yl]pyrrolidin-1-yl}-phenyl)-2-oxo-5-oxazolidinylmethyl] acetamide;
N-((5*S*)-3-{3-fluoro-4-[3(S)-(4-pyridin-2-yl-[1,2,3]triazol-2-yl)pyrrolidin-1-yl]-phenyl}-2-oxo-5-oxazolidinylmethyl) acetamide;
*N*-[(5*S*)-3-(3-fluoro-4-{3(*S*)-[4-(3-cyanophenyl)-[1,2,3]triazol-2-yl]pyrrolidin-1-yl}-phenyl)-2-oxo-5-oxazolidinylmethyl] acetamide;
*N*-{(5*S*)-3-(3, 5-difluoro-4-[3(*S*)-([1,2,3]triazol-2-yl pyrrolidin-1-yl) phenyl]-2-oxo-5-oxazolidinyl methyl} acetamide;
*N*-{(5*S*)-3-[3, 5-difluoro-4-[3(*S*)-([1,2,3]triazol-1-yl pyrrolidin-1-yl) phenyl]-2-oxo-5-oxazolidinyl methyl} acetamide;
*N*-{(5S)-3-[3, 5-difluoro-4-[3(*S*)-(tetrazol-2-yl pyrrolidin-1-yl) phenyl]-2-oxo-5-oxazolidinyl methyl} acetamide; and
*N*-{(5*S*)-3-[3, 5-difluoro-4-[3(*S*)-(tetrazol-1-yl pyrrolidin-1-yl) phenyl]-2-oxo-5-oxazolidinyl methyl} acetamide.

The compounds of general formula (I) may be prepared by
(i) a) reacting an intermediate of formula (II), 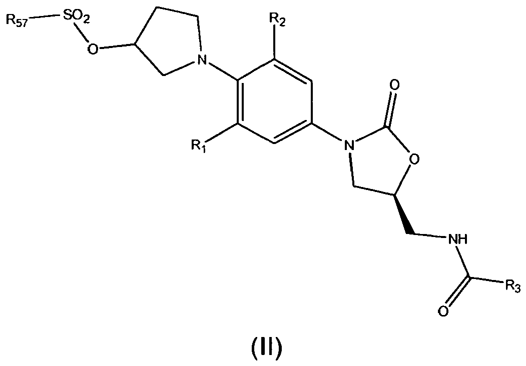 wherein Ri, R₂ and R₃ are as defined above, and R₅₇ is selected from methyl, phenyl, p-tolyl, p-bromophenyl, p-nitrophenyl, trifluoromethyl, and 2,2,2-trifluoroethyl, with an intermediate of formula RH (III), wherein R is as defined above, in an inert solvent and in the presence of a base; or b) reacting an intermediate of formula (IV), 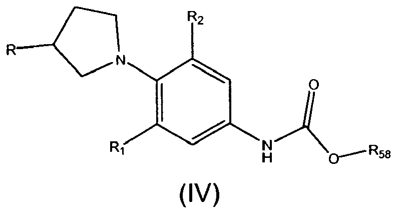 wherein R, R₁ and R₂ are as defined above and R₅₈ is selected from linear or branched (1-6C)alkyl, and benzyl optionally substituted in the phenyl ring by up to three linear or branched (1-6C)alkyl groups, with an intermediate of formula (V), 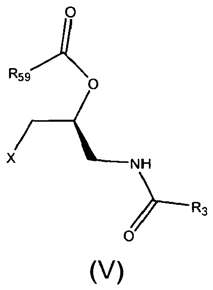 wherein R₃ is as defined above, R₅₉ is a linear or branched (1-6C)alkyl group, and X is a halogen atom, in an inert solvent and in the presence of a strong basic catalyst; and
(ii) recovering the resultant compound of formula (I) in free or pharmaceutically acceptable salt, solvate, hydrate, or enantiomeric form.

Preferably R₅₇ is methyl, R₅₈ is benzyl, R₅₉ is methyl, and X is bromine.

Inert solvents in step (ia) are preferably aprotic solvents. Suitable aprotic solvents are polar ethers such as, for example, tetrahydrofuran, methyltetrahydrofuran, dioxane, tert-butylmethylether, or dimethoxyethylether, or amides such as, for example, dimethylformamide, or lactams such as, for example, N-methylpyrrolidone, and mixtures thereof. Examples of bases include carbonates such as lithium carbonate, lithium bicarbonate, sodium carbonate, sodium bicarbonate, potassium carbonate, potassium bicarbonate, cesium carbonate, and the like, and mixtures thereof.

Inert solvents in step (ib) are preferably aprotic solvents. Suitable aprotic solvents are polar ethers such as, for example, tetrahydrofuran, methyltetrahydrofuran, dioxane, tert-butylmethylether, or dimethoxyethylether, or amides such as, for example, dimethylformamide, or lactams such as, for example, N-methylpyrrolidone, and mixtures thereof. Suitable solvents are also mixtures of such aprotic solvents and alcohols such as, for example, methanol or ethanol. Examples of strong basic catalysts include hydroxides such as lithium hydroxide, sodium hydroxide, and potassium hydroxide, alkoxides, such as lithium *tert*-butoxide, sodium *tert*-butoxide, and potassium *tert*-butoxide, alkyllithiums such as *tert*-butyllithium, n-butyllithium, and methyllithium, dialkylamides such as lithium diisopropylamide, disilylamides such as lithium hexamethyldisilazide, potassium hexamethyldisilazide, and sodium hexamethyldisilazide, and hydrides such as lithium hydride, sodium hydride, and potassium hydride.

Useful processes for recovering the resultant compounds in step (ii) include conventional methods known to the person skilled in the art such as crystallization and chromatographic processes, resolution of racemic forms by chromatographic separation using a chiral stationary phase, and also processes involving fractional crystallization. This can, in particular, involve the separation of individual enantiomers, for example, diastereoisomeric salts formed with chiral acids, for example (+)-tartaric acid, (-)-tartaric acid, or (+)-1 0-camphorsulfonic acid.

The compounds are useful antimicrobial agents, effective against a number of human and veterinary microorganisms. Some non limitative examples of these microorganisms are *Staphylococcus aureus, Streptococcus pneumoniae, Haemophylus influenzae, Bacteroides fragilis, Moraxella catarrhalis,* and *Enterococcus faecium.*

The compounds of the present invention can be normally formulated in accordance with standard pharmaceutical practice as a pharmaceutical composition.

The pharmaceutical compositions of this invention may be administered in standard manner for the disease condition that it is desired to treat, for example by oral, parenteral, inhalatory, rectal, transdermal or topical administration. For these purposes the compounds of this invention may be formulated by means known in the art in the form of, for example, tablets, capsules, syrups, aqueous or oily solutions or suspensions, emulsions, dispersible powders, inhalatory solutions, suppositories, ointments, creams, drops and sterile aqueous or oily solutions or suspensions for injection and the like. The pharmaceutical compositions may contain flavoring agents, sweeteners, etc. in suitable solid or liquid carriers or diluents, or in a suitable sterile media to form suspensions or solutions suitable for intravenous, subcutaneous or intramuscular injection. Such compositions typically contain from 1 to 40%, preferably 1 to 10% by weight of active compound, the remainder of the composition being pharmaceutically acceptable carriers, diluents, solvents and the like.

The compounds of formula (I) are administered in an amount of 0.1 to 100 mg/kg of body weight/day, preferably 1 to 50 mg/kg of body weight/day. The compounds and compositions of the present invention are useful in the treatment of conditions such as nosocomial pneumoniae, community acquired pneumoniae, caused by methicillin-resistant *Staphylococcus aureus* (MRSA), including concurrent bacteremia, penicillin resistance and sensitive *Streptococcus pneumoniae,* diabetic foot infections and skin and skin structure infections, and all other infections caused by bacteria sensitive to the compounds described in the invention. The compounds of the present invention are effective against a number of human or animal pathogens, clinical isolates, including vancomycin-resistant organisms, methicillin-resistant organisms, and LNZ-R organisms.

Throughout the description and claims the word "comprise" and variations of the word, such as "comprising", are not intended to exclude other technical features, additives, components, or steps. Additional objects, advantages and features of the invention will become apparent to those skilled in the art upon examination of the description or may be learned by practice of the invention. The following Examples are provided by way of illustration, and are not intended to be limiting of the present invention.

### EXAMPLES

### Example 1: N-{(5S)-3-[3-fluoro-4-(3-methylsulfonyloxy pyrrolidin-1-yl)-phenyl]-2-oxo-5-oxazolidinyl methyl} acetamide

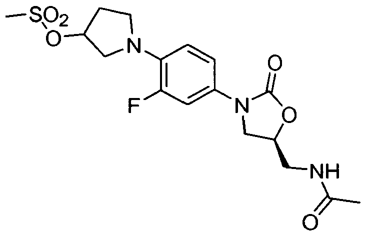

The *N*-{(5*S*)-3-[3-fluoro-4-(3-hydroxypyrrolidin-1-yl)-phenyl]-2-oxo-5-oxazolidinylmethyl}acetamide (2.8 g), prepared as described in WO 96/13502, and triethylamine (2.3 mL, 2 eq) were dissolved in dichloromethane (DCM) at room temperature and purged under argon. Methanesulfonyl chloride (0.9 mL, 1.5 eq) was added at 0°C and overnight at room temperature. Triethylamine and methanesulfonyl chloride were added to convert remaining alcohol. The reaction mixture was washed with water, brine and the organic layers dried over MgSO₄. The concentrated residue was purified by column chromatography (silica gel, DCM/MeOH increasing polarity) to afford 1.97 g of title compound.

HPLC (t, %): 6.53 min, 90%.

MS(ESI) *m*/*z* = 416(M+1)

¹H NMR (400 MHz, δ, ppm, DMSO): 2.21 (2H, m), 3.24 (3H, m), 3.35 (5H, m), 3.67 (2H, m), 4.05 (1H, t, J = 8 Hz), 4.67 (1H, m), 5.35 (1H, m), 6.80 (1H, t, J = 9.6 Hz), 7.11 (1H, dd, J = 2.4, 8.4 Hz), 7.43 ((1H, dd, J = 2.8, 16 Hz), 8.24 (1H, NH)

### Example 2: N-((5S)-3-{3-fluoro-4-[3-(2-triazolyl)pyrrolidin-1-yl]-phenyl}-2-oxo-5-oxazolidinyl methyl) acetamide

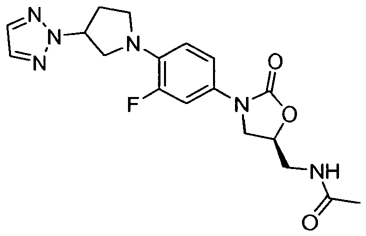

K₂CO₃ (0.6 mmol) and N-{(5S)-3-[3-fluoro-4-(3-methylsulfonyloxy pyrrolidin-1-yl)-phenyl]-2-oxo-5-oxazolidinyl methyl} acetamide (200 mg) were weighted and purged under argon in a 25 mL-round bottom flask. Dimethylformamide (DMF) and 1,2,3-triazole were added and the mixture refluxed at 70°C overnight. Cold water and DCM were added and the separated organic layer dried over MgSO₄ and concentrated under reduced pressure. The mixture of regioisomers was purified by column chromatography (silica gel, DCM/MeOH 95:5) to give 46 mg of the title compound as a major regioisomer (Yield = 25%).

HPLC (t, %): 6.8 min, 88%.

MS(ESI) *m*/*z* = 389 (M+1)

¹H NMR (400 MHz, δ, ppm, CDCl₃): 1.98 (3H, s), 2.55 (1H, m), 2.65 (1H, m), 3.62 (5H,m), 3.87 (1H, m), 3.97 (1H, m), 4.71 (1H, m), 5.31 (1H, m), 6.72 (1H, m), 6.98 (1H, m), 7.35 (1H, m), 7.59 (2H, s)

### Example 3: N-((5S)-3-{3-fluoro-4-[3-(1-triazolyl)pyrrolidin-1-yl]-phenyl}-2-oxo-5-oxazolidinyl methyl) acetamide

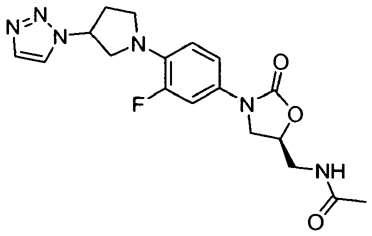

It was obtained concomitantly with compound of Example 2, which after column chromatography afforded 36 mg of title compound (Yield = 32%).

HPLC (t, %): 6.1 min, 99%.

MS(ESI) m/z = 389 (M+1)

¹H NMR (400 MHz, δ, ppm, DMSO): 1.82 (3H, s), 2.45 (1H, m), 2.55 (1H, m), 3.37 (3H,m), 3.55 (1H, M), 3.65 (2H, m), 3.85 (1H, m), 4.05 (1H, t, J = 8.8 Hz), 4.71 (1H, m), 5.38 (1H, m), 5.75 (1H, s), 6.83(1H, st, J = 10 Hz), 7.11 (1H, dd, J = 2.4, 9 Hz), 7.41 (1H, dd, J = 3, 16 Hz), 7.75(1H, s), 8.21 (1H, s), 8.22 (1H, NH)

### Example 4: N-((5S)-3-[3-fluoro-4-[3-(1-imidazolyl)pyrrolidin-1-yl]-phenyl}-2-oxo-5-oxazolidinyl methyl) acetamide

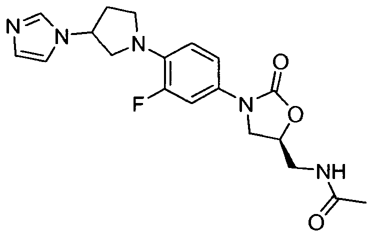

It was prepared following the same procedure as in Example 2, obtaining 28 mg of title compound purified by preparative HPLC.

HPLC (t, %): 6.18 min, 90%.

MS(ESI) *m*/*z* = 388 (M+1)

¹H NMR (400 MHz, δ, ppm, CDCl₃): 1.98 (3H, s), 2.20 (1H, m), 2.50 (1H, m), 3.37 (1H,m), 3.61 (6H, m), 3.98 (1H, t, J = 8.8 Hz), 4.71 (1H, m), 4.82 (1H, m), 6.66 (1H, st, J = 9.2 Hz), 7.01 (3H, m), 7.35 (1H, dd, J = 2.8, 15 Hz), 7.62(1H, s), 8.05 (1H, NH)

### Example 5: N-((5S)-3-{3-fluoro-4-[3-(2-tetrazolyl)pyrrolidinyl]-phenyl}-2-oxo-5-oxazolidinyl methyl) acetamide

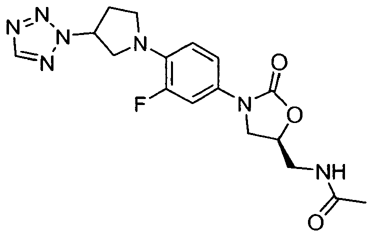

It was prepared following the same procedure as in Example 2, obtaining 45 mg of title compound purified by preparative HPLC.

HPLC (t, %): 6.53 min, 92%.

MS(ESI) *m*/z = 390 (M+1)

¹H NMR (400 MHz, δ, ppm, DMSO): 2.00 (3H, s), 2.66 (2H, m), 3.71 (7H, m), 4.73 (1H, m), 5.55 (1H, m), 5.96 (1H, m), 6.69 (1H, t, J = 10 Hz), 7.02 (1H, m), 7.36 (1H, m), 8.50(1H, s)

### Example 6: N-((5S)-3-{3-fluoro-4-[3-(1-tetrazolyl)pyrrolidinyl]-phenyl}-2-oxo-5-oxazolidinyl methyl) acetamide

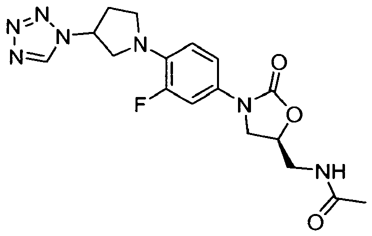

It was obtained concomitantly with compound of Example 5, which after HPLC purification afforded 11 mg of title compound.

HPLC (t, %): 6.10 min, 94%.

MS(ESI) *m*/*z* = 390 (M+1) ¹H NMR (400 MHz, δ, ppm, DMSO): 2.00 (3H, s), 2.39 (1H, m), 2.64 (1H, m), 3.55 (7H, m), 3.98 (1H, t, J = 8.8 Hz), 4.70 (1H, m), 5.39 (1H, m), 6.70 (1H, t, J = 9.6 Hz), 7.01 (1H, m), 7.36 (1H, dd, J = 15, 2.4 Hz), 8.77(1H, s)

### Example 7: N-{(5S)-3-[3-fluoro-4-(3-[1, 2, 4]triazol-1-yl-pyrrolidin-1-yl)-phenyl]-2-oxo-5-oxazolidinylmethyl} acetamide

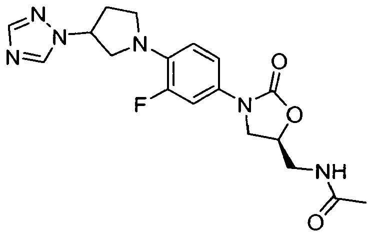

It was prepared following the same procedure as in Example 2, obtaining 27 mg of title compound.

HPLC (t, %): 5.99 min, 90%.

MS(ESI) *m*/*z* = 389 (M+1)

¹H NMR (400 MHz, δ, ppm, DMSO): 2.00 (3H, s), 2.49 (2H, m), 3.65 (7H, m), 3.98 (1H, t, J = 8.8 Hz), 4.74 (1H, m), 5.07 (1H, m), 6.36 (1H, NH), 6.67 (1H, t, J = 9.2 Hz), 7.05 (1H, m), 7.35 (1H, dd, J = 15, 2.8 Hz), 7.93 (1H, s), 8.18 (1H, s)

### Example 8: N-((5S)-3-{3-fluoro-4-[3-(benzotriazolyl)pyrrolidin-1-yl]-phenyl}-2-oxo-5-oxazolidinyl methyl) acetamide

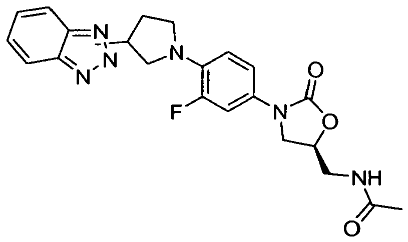

It was prepared following the same procedure as in Example 2, obtaining 71.5 mg of title compound as a mixture of the two possible regioisomers.

HPLC (t, %): 4.68 min, 37.7%; 5.08, 57.8%.

MS(ESI) *m*/*z* = 439 (M+1)

### Example 9: N-((5S)-3-{3-fluoro-4-[3-(4-nitropyrazolyl)pyrrolidinyl]-phenyl}-2-oxo-5-oxazolidinyl methyl) acetamide

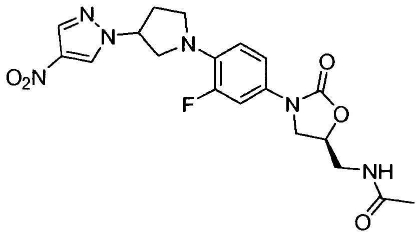

It was prepared following the same procedure as in Example 2, obtaining 49.1 mg of title compound.

HPLC (t, %): 4.68 min, 97%.

MS(ESI) *m*/*z* = 433 (M+1)

¹H NMR (400 MHz, δ, ppm, DMSO): 1.84 (3H, s), 2.45 (2H, m), 3.39 (3H, m), 3.58 (1H, m), 3.68 (2H, m), 3.79 (1H, m), 4.06 (1H, m), 4.69 (1H, m), 5.18 (1H, m), 6.84 (1H, t, J = 8 Hz), 7.12 (1H, d, J = 6.2 Hz), 7.42 (1H, d, J = 13 Hz), 8.23 (1H, NH), 8.32(1H, s), 8.99 (1H, s)

### Example 10: N-((5S)-3-{3-fluoro-4-[3-(5-p-tolyltetrazol-2-yl)pyrrolidin-1-yl]-phenyl}-2-oxo-5-oxazolidinylmethyl) acetamide

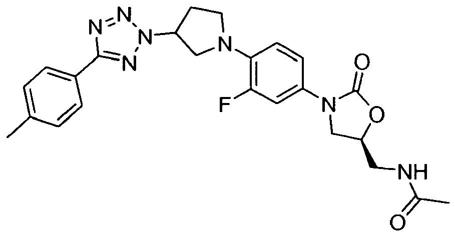

It was prepared following the same procedure as in Example 2, obtaining 41.1 mg of title compound.

HPLC (t, %): 5.45 min, 95%.

MS(ESI) m/z = 480 (M+1)

¹H NMR (400 MHz, δ, ppm, DMSO): 1.84 (3H, s), 2.38 (3H, s), 2.65 (2H, m), 3.40 (2H, m), 3.48 (1H, m), 3.67 (2H, m), 3.90 (1H, m), 3.97 (1H, m), 4.07 (1H, m), 4.69 (1H, m), 5.74 (1H, m), 6.88 (1H, t, J = 7.5 Hz), 7.13 (1H, d, J = 6.7 Hz), 7.37 (2H, d, J = 6.2 Hz), 7.42 (1H, d, J = 13 Hz), 7.95 (2H, d, J = 6.2 Hz), 8.24 (1H, NH)

### Example 11: N-((5S)-3-{3-fluoro-4-[3-(4-pyrimidin-4-yl-pyrazol-1-yl)pyrrolidin-1-yl]-phenyl}-2-oxo-5-oxazolidinylmethyl) acetamide

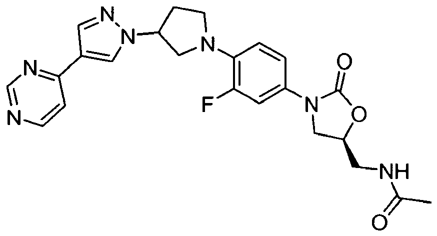

It was prepared following the same procedure as in Example 2, obtaining 61.7 mg of title compound.

HPLC (t, %): 4.08 min, 96%.

MS(ESI) *m*/*z* = 466 (M+1) ¹H NMR (400 MHz, δ, ppm, DMSO): 1.84 (3H, s), 2.45 (2H, m), 3.39 (3H, m), 3.59 (1H, m), 3.68 (1H, m), 3.81 (2H, m), 4.06 (1H, m), 4.69 (1H, m), 5.18 (1H, m), 6.85 (1H, t, J = 7.5 Hz), 7.12 (1H, d, J = 6.7 Hz), 7.43 (1H, d, J = 12.5 Hz), 7.76 (1H, d, J = 4 Hz), 8.23 (2H, m), 8.64 (1H, s), 8.69 (1H, d, J = 4 Hz), 9.05 (1H, s)

### Example 12: N-((5S)-3-{3-fluoro-4-[3-(4-pyrazin-2-yl-pyrazol-1-yl)pyrrolidin-1-yl]-phenyl}-2-oxo-5-oxazolidinylmethyl) acetamide

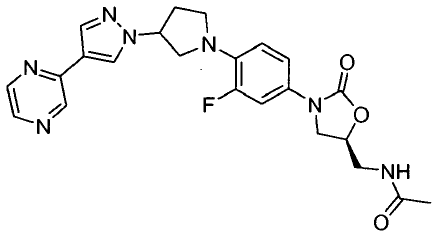

It was prepared following the same procedure as in Example 2, obtaining 62.1 mg of title compound.

HPLC (t, %): 4.20 min, 98%.

MS(ESI) *m*/*z* = 466 (M+1)

¹H NMR (400 MHz, δ, ppm, DMSO): 1.84 (3H, s), 2.45 (2H, m), 3.39 (3H, m), 3.59 (1H, m), 3.68 (1H, m), 3.81 (2H, m), 4.06 (1H, m), 4.69 (1H, m), 5.18 (1H, m), 6.85 (1H, t, J = 7.5 Hz), 7.12 (1H, d, J = 6.7 Hz), 7.43 (1H, d, J = 12.5 Hz), 8.17 (1H, s), 8.24 (1H, m), 8.42 (1H, s), 8.55 (1H, s), 8.58 (1H, s), 9.01 (1H, s)

### Example 13: N-((5S)-3-{3-fluoro-4-[3-(5-phenyltetrazol-2-yl)pyrrolidin-1-yl]-phenyl}-2-oxo-5-oxazolidinylmethyl) acetamide

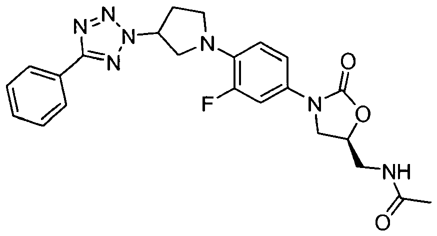

It was prepared following the same procedure as in Example 2, obtaining 61.6 mg of title compound.

HPLC (t, %): 5.22 min, 96%.

MS(ESI) *m*/*z* = 466 (M+1)

¹H NMR (400 MHz, δ, ppm, DMSO): 1.84 (3H, s), 2.65 (2H, m), 3.40 (2H, m), 3.48 (1H, m), 3.67 (2H, m), 3.90 (1H, m), 3.97 (1H, m), 4.07 (1H, m), 4.69 (1H, m), 5.76 (1H, m), 6.88 (1H, t, J = 7.5 Hz), 7.13 (1H, d, J = 6.7 Hz), 7.44 (1H, d, J = 13 Hz), 7.56 (3H, m), 8.06 (2H, m), 8.24 (1H, NH)

### Example 14: N-((5S)-3-{3-fluoro-4-[3-(5-methylsulfanylltetrazol-2-yl)pyrrolidin-1-yl]-phenyl}-2-oxo-5-oxazolidinylmethyl) acetamide

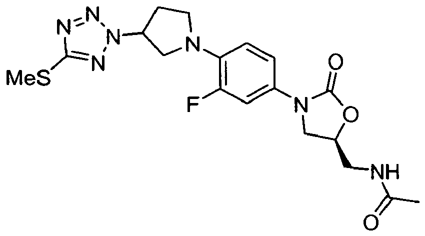

It was prepared following the same procedure as in Example 2, obtaining 55 mg of title compound.

HPLC (t, %): 4.72 min, 98%.

MS(ESI) *m*/*z* = 436 (M+1) ¹H NMR (400 MHz, δ, ppm, CDCl₃): 2.02 (3H, s), 2.65 (2H, m), 2,66 (3H, s), 3.59 (2H, m), 3.70 (3H, m), 3.90 (1H, m), 4.02 (2H, m), 4.75 (1H, m), 5.45 (1H, m), 6.68 (1H, t, J = 7.4 Hz), 7.03 (1H, m), 7.36 (1H, d, J = 12 Hz)

### Example 15: N-((5S)-3-{3-fluoro-4-[3-(5-thiophen-2-yl-tetrazol-2-yl)pyrrolidin-1-yl]-phenyl}-2-oxo-5-oxazolidinylmethyl) acetamide.

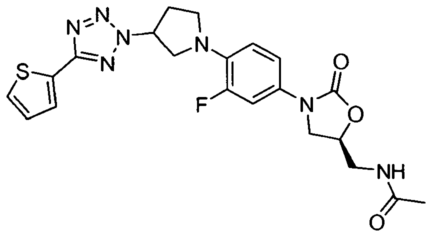

It was prepared following the same procedure as in Example 2, obtaining 50 mg of title compound.

HPLC (t, %): 5.07 min, 97%.

MS(ESI) *m*/*z* = 472 (M+1)

¹H NMR (400 MHz, δ, ppm, CDCl₃): 2.02 (3H, s), 2.65 (1H, m), 2,80 (1H, m), 3.60 (2H, m), 3.72 (3H, m), 3.97 (2H, m), 4.08 (1H, m), 4.74 (1H, m), 5.53 (1H, m), 5.96 (1H, m), 6.71 (1H, t, J = 7.5Hz), 7.04 (1H, m), 7.15 (1H, m), 7.37 (1H, d, J = 12 Hz), 7.45 (1H, m), 7.79 (1H, s)

### Example 16: N-((5S)-3-{3-fluoro-4-[3-(5-methyltetrazol-2-yl)pyrrolidin-1-yl]-phenyl}-2-oxo-5-oxazolidinylmethyl) acetamide

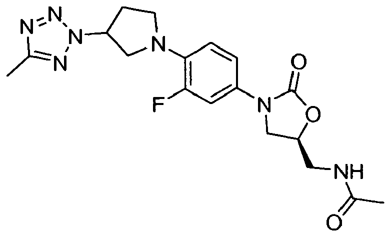

It was prepared following the same procedure as in Example 2, obtaining 36.6 mg of title compound.

HPLC (t, %): 4.25 min, 95%.

MS(ESI) *m*/*z* = 404 (M+1)

¹H NMR (400 MHz, δ, ppm, CDCl₃): 2.02 (3H, s), 2.53 (3H, s), 2,61 (2H, m), 3.58 (2H, m), 3.70 (3H, m), 3.90 (1H, m), 4.02 (2H, m), 4.75 (1H, m), 5.46 (1H, m), 5.96 (1H, m), 6.68 (1H, t, J = 7.4 Hz), 7.03 (1H, d, J = 8 Hz), 7.36 (1H, d, J = 11 Hz)

### Example 17: N-((5S)-3-{3-fluoro-4-[3-(4-bromopyrazol-1-yl)pyrrolidin-1-yl]-phenyl}-2-oxo-5-oxazolidinylmethyl) acetamide

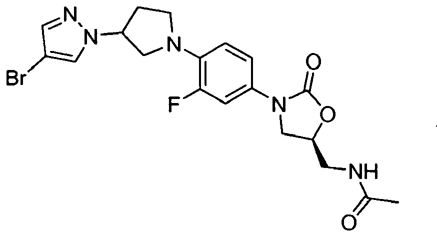

It was prepared following the same procedure as in Example 2, obtaining 51.7 mg of title compound.

HPLC (t, %): 4.92 min, 97%.

MS(ESI) *m*/*z* = 466-468 (M+1)

¹H NMR (400 MHz, δ, ppm, DMSO): 1.84 (3H, s), 2.45 (2H, m), 3.39 (3H, m), 3.54 (1H, m), 3.60 (1H, m), 3.68 (1H, m), 3.75 (1H, m), 4.06 (1H, m), 4.69 (1H, m), 5.08 (1H, m), 6.84 (1H, t, J = 8 Hz), 7.12 (1H, d, J = 6.2 Hz), 7.42 (1H, d, J = 13 Hz), 7.59 (1H, s), 8.11 (1H, s), 8.23 (1H, NH)

### Example 18: N-((5S)-3-{3-fluoro-4-[3-(4-pyridin-4-yl-pyrazol-1-yl)pyrrolidin-1-yl]-phenyl}-2-oxo-5-oxazolidinylmethyl) acetamide

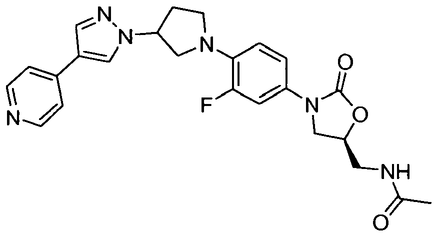

It was prepared following the same procedure as in Example 2, obtaining 61.6 mg of title compound.

HPLC (t, %): 3.50 min, 96%.

MS(ESI) *m*/*z* = 465 (M+1)

¹H NMR (400 MHz, δ, ppm, DMSO): 1.84 (3H, s), 2.45 (2H, m), 3.42 (3H, m), 3.59 (1H, m), 3.69 (1H, m), 3.81 (2H, m), 4.06 (1H, m), 4.69 (1H, m), 5.12 (1H, m), 6.85 (1H, t, J = 7.5 Hz), 7.12 (1H, d, J = 6.7 Hz), 7.42 (1H, d, J = 12.5 Hz), 7.59 (2H, m), 8.12 (1H, s), 8.24 (1H, NH), 8.49 (1H, m), 8.56 (1H, s)

### Example 19: N-[(5S)-3-(3-fluoro-4-{3-[4-(4-nitrophenyl)-pyrazol-1-yl]pyrrolidin-1-yl}-phenyl)-2-oxo-5-oxazolidinylmethyl] acetamide

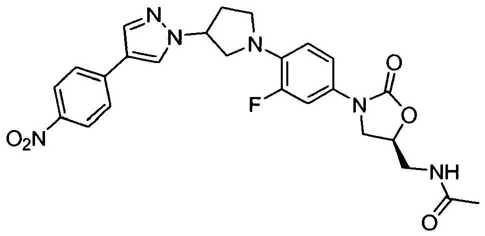

It was prepared following the same procedure as in Example 2, obtaining 52.2 mg of title compound.

HPLC (t, %): 5.20 min, 99%.

MS(ESI) *m*/*z* = 509 (M+1)

¹H NMR (400 MHz, δ, ppm, DMSO): 1.84 (3H, s), 2.45 (2H, m), 3.39 (5H, m), 3.59 (1H, m), 3.69 (1H, m), 3.81 (2H, m), 4.06 (1H, m), 4.69 (1H, m), 5.13 (1H, m), 6.85 (1H, t, J = 7.5 Hz), 7.12 (1H, d, J = 6.7 Hz), 7.42 (1H, d, J = 12.5 Hz), 7.89 (2H, m), 8.15 (1H, s), 8.24 (3H, m), 8.59 (1H, s)

### Example 20: N-[(5S)-3-(3-fluoro-4-{3-[4-(2-trifluoromethyl-phenyl)-pyrazol-1-yl]pyrrolidin-1-yl}-phenyl)-2-oxo-5-oxazolidinylmethyl] acetamide

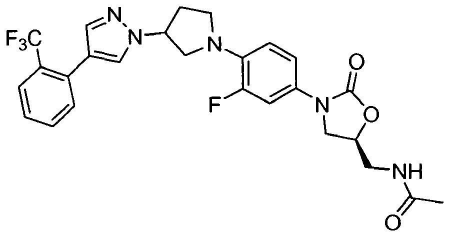

It was prepared following the same procedure as in Example 2, obtaining 55 mg of title compound.

HPLC (t, %): 5.62 min, 99%.

MS(ESI) *m*/*z* = 532 (M+1)

¹H NMR (400 MHz, δ, ppm, CDCl₃): 2.02 (3H, s), 2.53 (2H, m), 2.50 (1H, m), 3.55 (2H,m), 3.70 (5H, m), 3.82 (1H, m), 3.89 (1H, m), 4.01 (1H, m), 4.75 (1H, m), 5.08 (1H, m), 6.03 (1H, NH), 6.47 (1H, s), 6.71 (1H, m), 7.03 (1H, d, J = 7 Hz), 7.37 (1H, m), 7.45 (1H, m), 7.54 (1H, m), 7.66 (1H, m), 7.73 (1H, m)

### Example 21: N-[(5S)-3-(3-fluoro-4-{3-[4-(2-methoxyphenyl)-pyrazol-1-yl]pyrrolidin-1-yl}-phenyl)-2-oxo-5-oxazolidinylmethyl] acetamide

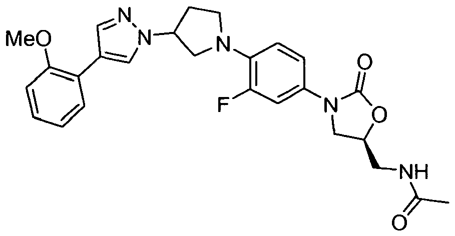

It was prepared following the same procedure as in Example 2, obtaining 21.6 mg of title compound.

HPLC (t, %): 5.28 min, 96%.

MS(ESI) *m*/*z* = 494 (M+1)

¹H NMR (400 MHz, δ, ppm, CDCl₃): 2.02 (3H, s), 2.52 (2H, m), 3.55 (2H,m), 3.70 (4H, m), 3.87 (1H, m), 3.88 (3H, s), 3.99 (1H, m), 4.01 (1H, m), 4.75 (1H, m), 5.08 (1H, m), 5.98 (1H, NH), 6.70 (1H, m), 6.78 (1H, s), 7.00 (3H, m), 7.37 (1H, m), 7.52 (1H, s), 7.91 (1H, d, J = 6.4 Hz)

### Example 22: N-((5S)-3-{3-fluoro-4-[3-(4-acetylpyrazol-1-yl)pyrrolidin-1-yl]-phenyl}-2-oxo-5-oxazolidinylmethyl) acetamide

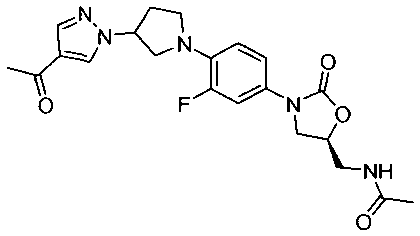

It was prepared following the same procedure as in Example 2, obtaining 34.6 mg of title compound.

HPLC (t, %): 8.38 min, 96%.

MS(ESI) *m*/*z* = 430 (M+1) ¹H NMR (400 MHz, δ, ppm, CDCl₃):2.02 (3H, s), 2.47 (1H, m), 2.56 (4H, s), 3.48 (1H, m), 3.60 (1H, m), 3.70 (3H, m), 3.85 (2H, m), 4.01 (1H, m), 4.75 (1H, m), 5.08 (1H, m), 5.99 (1H, m), 6.71 (1H, t, J = 7.6 Hz), 6.79 (1H, s), 7.05 (1H, d, J = 6.8), 7.38 (1H, d, J = 12 Hz), 7.52 (1H, s)

### Example 23: N-((5S)-3-{3-fluoro-4-[3-(3-phenylpyrazol-1-yl)pyrrolidin-1-yl]-phenyl}-2-oxo-5-oxazolidinylmethyl) acetamide

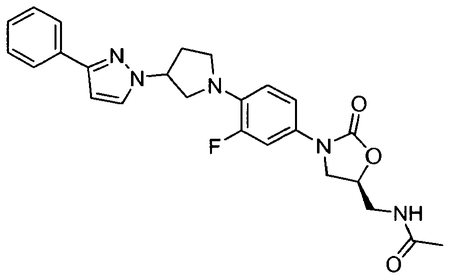

It was prepared following the same procedure as in Example 2, obtaining 43.7 mg of title compound.

HPLC (t, %): 5.35 min, 96%.

MS(ESI) *m*/*z* = 464 (M+1)

¹H NMR (400 MHz, δ, ppm, CDCl₃): 2.02 (3H, s), 2.52 (2H, m), 3.55 (2H, m), 3.73 (3H, m), 3.85 (2H, m), 4.01 (1H, m), 4.75 (1H, m), 5.08 (1H, m), 5.99 (1H, m), 6.56 (1H, s), 6.71 (1H, t, J = 7.3 Hz), 7.04 (1H, d, J = 6 Hz), 7.26 (1H, m), 7.38 (3H, m), 7.52 (1H, s), 7.79 (1H, d, J = 6 Hz)

### Example 24: N-[(5S)-3-(3-fluoro-4-{3-[4-(4-fluorophenyl)-pyrazol-1 - yl]pyrrolidin-1-yl)-phenyl)-2-oxo-5-oxazolidinylmethyl] acetamide

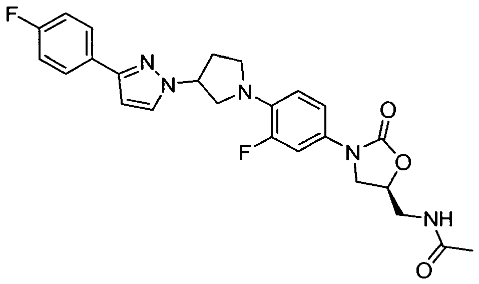

It was prepared following the same procedure as in Example 2, obtaining 48.7 mg of title compound.

HPLC (t, %): 5.42 min, 99%.

MS(ESI) *m*/*z* = 482 (M+1)

¹H NMR (400 MHz, δ, ppm, CDCl₃): 2.02 (3H, s), 2.50 (2H, m), 3.55 (2H, m), 3.71 (3H, m), 3.83 (2H, m), 4.01 (1H, t, J = 7 Hz), 4.75 (1H, m), 5.06 (1H, m), 5.96 (1H, m), 6.51 (1H, s), 6.71 (1H, t, J = 7.6 Hz), 7.05 (3H, m), 7.37 (1H, d, J = 13 Hz), 7.51 (1H, s), 7.76 (1H, m)

### Example 25: N-[(5S)-3-(3-fluoro-4-{3-[3-(2-fluorophenyl)-pyrazol-1-yl]pyrrolidin-1-yl}-phenyl)-2-oxo-5-oxazolidinylmethyl] acetamide

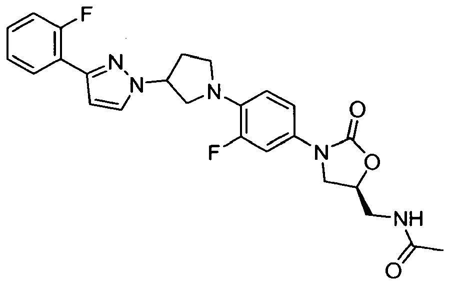

It was prepared following the same procedure as in Example 2, obtaining 26 mg of title compound.

HPLC (t, %): 5.42 min, 99%.

MS(ESI) *m*/*z* = 482 (M+1)

¹H NMR (400 MHz, δ, ppm, CDCl₃): 2.02 (3H, s), 2.50 (2H, m), 3.55 (2H, m), 3.71 (3H, m), 3.83 (2H, m), 4.01 (1H, t, J = 7 Hz), 4.75 (1H, m), 5.06 (1H, m), 5.96 (1H, m), 6.71 (2H, m), 7.10 (3H, m), 7.37 (1H, d, J = 13 Hz), 7.51 (1H, s), 7.98 (1H, m)

### Example 26: N-((5S)-3-{3-fluoro-4-[3-(3-trifluoromethyl-pyrazol-1-yl)pyrrolidin-1-yl]-phenyl}-2-oxo-5-oxazolidinylmethyl) acetamide

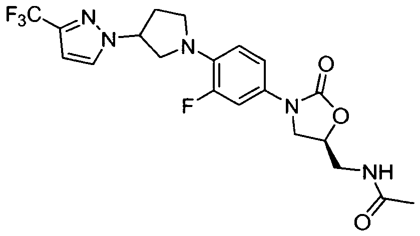

It was prepared following the same procedure as in Example 2, obtaining 62.2 mg of title compound.

HPLC (t, %): 5.18 min, 99%.

MS(ESI) *m*/*z* = 456 (M+1)

¹H NMR (400 MHz, δ, ppm, CDCl₃): 2.02 (3H, s), 2.50 (2H, m), 3.47 (1H, m), 3.59 (1H, m), 3.70 (4H, m), 4.02 (1H, t, J = 7 Hz), 4.75 (1H, m), 5.09 (1H, m), 5.96 (1H, m), 6.53 (1H, s), 6.70 (1H, t, J = 7.4 Hz), 7.04 (1H, d, J = 6 Hz), 7.39 (1H, d, J = 12 Hz), 7.57 (1H, s)

### Example 27: N-[(5S)-3-(3-fluoro-4-{3-[3-(4-trifluoromethyl-phenyl)-pyrazol-1-yl]pyrrolidin-1-yl}-phenyl)-2-oxo-5-oxazolidinylmethyl] acetamide

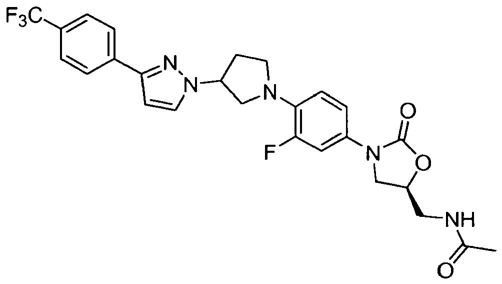

It was prepared following the same procedure as in Example 2, obtaining 37.2 mg of title compound.

HPLC (t, %): 5.83 min, 98%.

MS(ESI) *m*/*z* = 532 (M+1)

¹H NMR (400 MHz, δ, ppm, CDCl₃): 2.02 (3H, s), 2.52 (2H, m), 3.55 (2H,m), 3.70 (4H, m), 3.87 (1H, m), 3.88 (3H, s), 3.99 (1H, m), 4.01 (1H, m), 4.75 (1H, m), 5.08 (1H, m), 5.98 (1H, NH), 6.60 (1H, s), 6.72 (1H, t, J = 7.5 Hz), 7.05 (1H, d, J = 7 Hz), 7.38 (1H, d, J = 12 Hz), 7.55 (1H, s), 7.63 (2H, d, J = 7 Hz), 7.89 (2H, d, J = 6 Hz)

### Example 28: N-((5S)-3-{3-fluoro-4-[3-(4-pyridin-2-yl-[1,2,3]triazol-2-yl)pyrrolidin-1-yl]-phenyl}-2-oxo-5-oxazolidinylmethyl) acetamide.

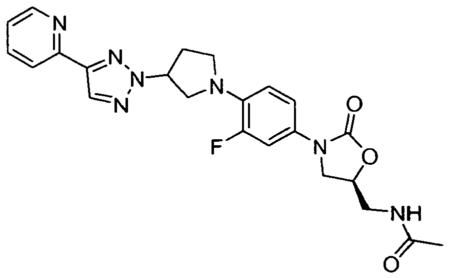

It was prepared following the same procedure as in Example 2, obtaining 76.6 mg of title compound as a mixture of the two possible regioisomers (only major shown).

HPLC (t, %): 4.15 min, 16%; 4.57 min, 82%.

MS(ESI) *m*/*z* = 466 (M+1)

### Example 29: N-[(5S)-3-(3-fluoro-4-{3-[4-(3-cyanophenyl)-[1,2,3]triazol-2-yl]pyrrolidin-1-yl}-phenyl)-2-oxo-5-oxazolidinylmethyl] acetamide

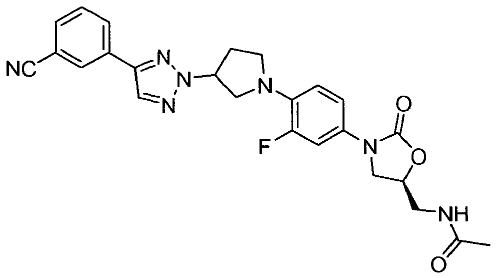

It was prepared following the same procedure as in Example 2, obtaining 64.7 mg of title compound as a mixture of the two possible regioisomers (only major shown).

HPLC (t, %): 4.85 min, 19%; 5.27 min, 77%.

MS(ESI) *m*/*z* = 490 (M+1)

### Example 30: 3, 5-difluoro-4-(3-hydroxy pyrrolidin-1-yl)-4-nitrobenzene

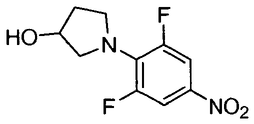

3-pyrrolidinol (2 mL, 24.7 mmol) and potassium carbonate (4.8 g) were dissolved in DMF (5 mL), and 3,4,5-trifluoronitrobenzene (2.6 mL) was slowly added and stirred under argon at room temperature overnight. The mixture was treated with cold water and the product was separated as a solid. This solid was filtered, washed with water and dried at 60°C under vacuum to give 5.4 g (Yield = 89%) of title compound.

HPLC (t, %): 7.71 min, 100 %.

MS(ESI) *m*/*z* = 245 (M+1)

¹H NMR (400 MHz, δ, ppm, DMSO): 1.87 (2H, m), 3.45 (1H, m), 3.69 (1H, m), 3.85 (2H, m), 4.33 (1H, m), 5.05 (1H, m), 7.86(2H, dd, J = 2.8, 9.6 Hz)

### Example 31: 3, 5-difluoro-4-(3-methylsulfonyloxy pyrrolidin-1-yl)-nitrobenzene

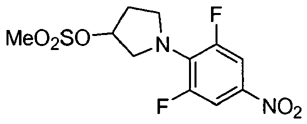

3, 5-difluoro-4-(3-hydroxy pyrrolidinyl)-nitrobenzene (5.4 g) and triethylamine (6.1 mL) were dissolved in DCM (125 mL) at room temperature and purged under argon. Methanesulfonyl chloride (2.6 mL) was added at 0°C and over night at room temperature. The reaction mixture was washed with water, brine and the organic layers dried over MgSO₄. The concentrated residue was transferred to a mortar and dried under vacuum at 55°C for 3 hours to afford 6.4 g of title compound.

HPLC (t, %): 8.32 min, 100 %.

MS(ESI) m/z = 323 (M+1)

¹H NMR (400 MHz, δ, ppm, DMSO): 2.2 (2H, m), 3.26 (3H, s), 3.83 (3H, m), 4.09 (1H, m), 5.38 (1H, s), 7.90 (2H, dd, J = 2.8, 9.2 Hz)

### Example 32: 3, 5-difluoro-4-[3-([1,2,3]-triazol-2-yl)pyrrolidinyl]-nitrobenzene

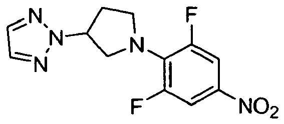

K₂CO₃ (322 mg) and 3, 5-difluoro-4-(3-methylsulfonyloxy pyrrolidinyl)-nitrobenzene (500 mg) were weighted and purged under argon in a 25 mL-round bottom flask. DMF (10 mL) and triazol (0.135 mL) were added and the mixture refluxed at 70°C overnight. Cold water and DCM were added and the separated organic layer dried over MgSO₄ and concentrated under reduced pressure. The mixture of regioisomers was purified by column chromatography (silica gel, DCM/MeOH 95:5) to give 274 mg of the title compound as a major regioisomer (Yield = 60%).

HPLC (t, %): 8.79 min, 100%.

MS(ESI) *m*/*z* = 296 (M+1)

¹H NMR (400 MHz, δ, ppm, DMSO): 2.49 (2H, m), 3.88 (2H, m), 4.11 (1H, m), 4.27 (1H, m), 5.37 (1H, m), 7.83 (2H, s), 7.90 (2H, dd, J = 2, 9.2 Hz)

### Example 33: 3, 5-difluoro-4-[3-([1,2,3]-triazol-1-yl)pyrrolidinyl]-nitrobenzene

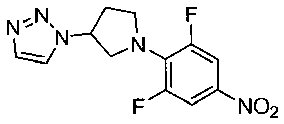

It was obtained concomitantly with compound of Example 32, which after column chromatography afforded 72 mg of title compound (Yield = 15%).

HPLC (t, %): 7.72 min, 80%.

MS(ESI) *m*/*z* = 296 (M+1)

¹H NMR (400 MHz, δ, ppm, DMSO): 2.49 (2H, m), 3.88 (2H, m), 4.08 (1H, m), 4.27 (1H, m), 5.36 (1H, m), 7.76 (1H, s), 7.92 (2H, dd, J = 2.4, 9.2 Hz), 8.28 (1H, s)

### Example 34: 3, 5-difluoro-4-[3-(tetrazol-2-yl) pyrrolidin-1-yl]-nitrobenzene

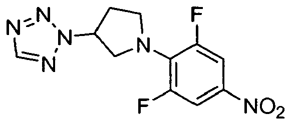

K₂CO₃ (322 mg) and 3, 5-difluoro-4-(3-methylsulfonyloxy pyrrolidinyl)-nitrobenzene (500 mg) were weighted and purged under argon in a 100 mL-round bottom flask. DMF (10 mL) and tetrazole solution (3% wt. in acetonitrile, 13.76 mL) were added and the mixture refluxed at 100°C overnight when complete reaction was observed by HPLC-MS. The reaction mixture was poured into crushed ice and the separated solid was washed with cold water to give 278 mg of a mixture of regioisomers. The aqueous organic layers were extracted with DCM and the separated organic layer dried over MgSO₄ and concentrated under reduced pressure recovering 100 mg of the mixture of regioisomers. The crude compounds as a mixture of regioisomers were purified by column chromatography (silica gel, DCM/EtOAc increasing polarity) to give 180 mg of the title compound as a major regioisomer (Yield = 40%).

HPLC (t, %): 8.34 min, 100%.

MS(ESI) *m*/*z* = 297 (M+1)

¹H NMR (400 MHz, δ, ppm, DMSO): 2.56 (2H, m), 3.91 (2H, m), 4.15 (1H, m), 4.36 (1H, m), 5.73 (1H, m), 7.92 (2H, dd, J = 2.4, 9.2 Hz), 9.01 (1H, s)

### Example 35: 3, 5-difluoro-4-[3-(tetrazol-1-yl) pyrrolidin-1-yl]-nitrobenzene

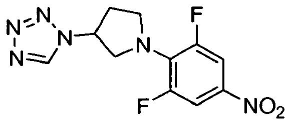

It was obtained concomitantly with compound of Example 34, which after column chromatography afforded 30 mg of title compound (Yield = 15%).

HPLC (t, %): 7.72 min, 96%.

MS(ESI) *m*/*z* = 297 (M+1)

¹H NMR (400 MHz, δ, ppm, DMSO): 2.56 (2H, m), 3.88 (2H, m), 4.10 (1H, m), 4.31 (1H, m), 5.45 (1H, m), 7.92 (2H, dd, J = 2.4, 9.2 Hz), 9.55 (1H, s)

### Example 36: N-{(5S)-3-[3, 5-difluoro-4-[3-(tetrazol-2-yl pyrrolidin-1-yl) phenyl]-2-oxo-5-oxazolidinyl methyl} acetamide

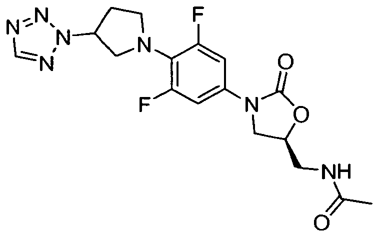

### 3, 5-difluoro-4-[3-(tetrazol-2-yl pyrrolidin-1-yl)]-phenylamine:

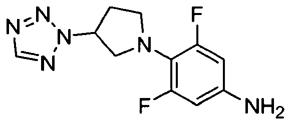

3,5-difluoro-4-[3-(tetrazol-2-ylpyrrolidin-1-yl)]- nitrobenzene (4 g) was dissolved in methanol (300 mL) and minimum quantity of DCM. The mixture was hydrogenated in a continuous-flow hydrogenation reactor using hydrogen generated in-situ from the electrolysis of water. First, the substrate is combined at room temperature with hydrogen at atmospheric pressure. And then, the mixture was passed through a packed Pd on Carbon (10%) cartridge where the reaction takes place. The product eluted out of the cartridge and into a collection vial to give after concentration 3.6 g of compound of 99% purity (100% Yield).

HPLC (t, %): 7.41 min, 98%.

MS(ESI) *m*/*z* = 267 (M+1).

¹H NMR (400 MHz, ppm, DMSO): 2.54 (2H, m), 3.26 (1H, m), 3.42 (1H, m), 3.54 (1H, m), 3.75 (1H, m), 5.59 (1H, m), 6.18 (2H, d, J = 12 Hz), 8.97 (1H, s).

### 3,5-difluoro-4-[3-(tetrazol-2-yl pyrrolidin-1-yl)]-phenyl carbamic acid benzyl ester:

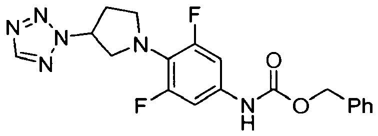

3,5-difluoro-4-[3-(tetrazol-2-ylpyrrolidin-1-yl)]-phenylamine (3.6 g) was dissolved in acetone (100 mL) and cooled to 0°C. Sodium hydrogencarbonate (4.6 g, 4 eq) in water (50 mL) was added, followed by benzyl chloroformate (3.9 mL, 2 eq) over 30 minutes. The mixture was stirred and the temperature allowed rise to ambient over 12 hours. DCM was added and the organic layer separated, and washed with water and brine. The combined organic layers were dried over magnesium sulfate and concentrated. The residue was purified by column chromatography over silica eluting with DCM and DCM/AcOEt from 0 to 10% to give 6.29 g of title product (94% Yield).

HPLC (t, %): 9.71 min, 94%.

MS(ESI) *m*/*z* = 401 (M+1).

¹H NMR (400 MHz, ppm, DMSO): 2.54 (2H, m), 3.4 (2H, m), 3.61 (1H, m), 3.73 (1H, m), 3.94 (1H, m), 5.13 (2H, s), 5.64 (1H, m), 7.09 (2H, d, J = 12 Hz), 7.38 (5H, m), 8.99 (1H, s).

### N-{(5S)-3-[3, 5-difluoro-4-[3-(tetrazol-2-yl pyrrolidin-1-yl) phenyl]-2-oxo-5-oxazolidinyl methyl} acetamide.

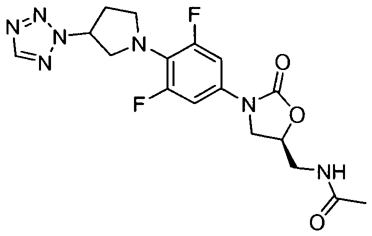

To a solution of 3.5 g of 3,5-difluoro-4-[3-(tetrazol-2-yl pyrrolidin-1-yl)]-phenyl carbamic acid benzyl ester in 5 mL of DMF was added 22 mL of a solution of 1 M of lithium *tert*-butoxide in THF and stirred at room temperature for 30 minutes. 0.6 mL of methanol and a solution of 3.5 g of (S)-*N*-(3-bromo-2-acetoxypropyl)acetamide in 5 mL of DMF were added and allowed to stand at room temperature for two days at which point HPLC showed nearly complete conversion. DCM and saturated aqueous ammonium chloride were added to the reaction solution and the separated organic layer was washed with water, brine and dried over anhydrous magnesium sulfate. The solvent was evaporated and the residue was purified by silica gel column chromatography (DCM/AcOEt from 0 to 10% and DCM/Methanol at 10%) to afford 2.6 g of the title compound (86% Yield).

HPLC (t, %): 6.95, 100.

MS(ESI) *m*/*z* = 407 (M+1)

¹H NMR (400 MHz, ppm, DMSO): 1.82 (3H, s), 2.55 (2H, m), 3.39 (2H, m), 3.51 (1H, m), 3.67 (2H, m), 3.81 (1H, m), 4.02 (2H, m), 4.71 (1H, m), 5.66 (1H, m), 7.22 (2H, d, J = 12 Hz), 8.24 (1H, NH), 8.99 (1H, s).

### Example 37: N-{(5S)-3-[3, 5-difluoro-4-[3-([1,2,3]triazol-2-yl pyrrolidin-1-yl) phenyl]-2-oxo-5-oxazolidinyl methyl} acetamide

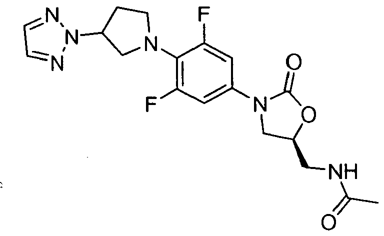

It was obtained from compound of Example 32 following procedure described in Example 36 to give after column chromatography 2.6 g of title compound.

HPLC (t, %): 7.22 min, 99%.

MS(ESI) *m*/*z* = 407 (M+1)

¹H NMR (400 MHz, δ, ppm, DMSO): 1.82 (3H, s), 2.49 (2H, m), 3.50 (1H, m), 3.65 (2H, m), 3.76 (1H, m), 3.92 (1H, m), 4.04 (1H, t, J = 8.8 Hz), 4.69 (1H, m), 5.31 (1H, q, J = 4.4 Hz), 7.20 (2H, d, J = 12 Hz), 7.81 (2H, s), 8.22 (1H, NH)

### Example 38: N-{(5S)-3-[3, 5-difluoro-4-[3-([1,2,3]triazol-1-yl pyrrolidin-1-yl) phenyl]-2-oxo-5-oxazolidinyl methyl} acetamide

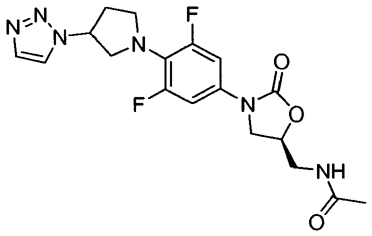

It was obtained from compound of Example 33 following procedure described in Example 36 to give after column chromatography 1.29 g of title compound.

HPLC (t, %): 6.52 min, 97%.

MS(ESI) m/*z* = 407 (M+1)

¹H NMR (400 MHz, δ, ppm, DMSO): 1.82 (3H, s), 2.35 (1H, m), 2.55 (1H, m), 3.83 (2H, m), 3.65 (2H, m), 3.88 (1H, m), 4.05 (1H, t, J = 8 Hz), 4.70 (1H, m), 5.33 (1H, m), 7.22 (2H, d, J = 12 Hz), 7.75 (2H, d, J = 0.8 Hz), 8.19 (1H, d, J = 1.2 Hz), 8.22 (1H, NH)

### Example 39: N-{(5S)-3-[3, 5-difluoro-4-[3-(tetrazol-1-yl pyrrolidin-1-yl) phenyl]-2-oxo-5-oxazolidinyl methyl} acetamide

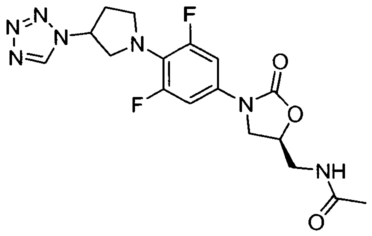

It was obtained from compound of Example 35 following procedure described in Example 36 to give after column chromatography 0.5 g of title compound.

HPLC (t, %): 6.48 min, 98%.

MS(ESI) *m*/*z* = 408 (M+1)

¹H NMR (400 MHz, δ, ppm, DMSO): 1.82 (3H, s), 2.41 (1H, m), 2.56 (1H, m), 3.34 (2H, m), 3.62 (4H, m), 3.87 (1H, m), 4.00 (1H, t, J = 9.2 Hz), 4.71 (1H, m), 5.40 (1H, m), 7.23 (2H, d, J = 12 Hz), 8.23 (1H, NH), 9.48 (1H, s)

### Example 40: Antibacterial activity

MICs were determined by using a standard micro dilution method according to The National Committee for Clinical Laboratory Standards (NCCLS), 5^{th} Approved standard M7-A5, 2001, Wayne, PA, USA. All compounds were tested against Gram-positive and Gram-negative bacteria showing relevant different susceptibility and resistance specifications. The used microorganisms were selected from laboratory reference bacteria and from clinical isolates.The tested concentrations were double dilutions from 0.06 µg/mL to 128 µg/mL in 96-well micro titter plates.

MICs were determined in the *Brucella* Blood medium supplemented for the anaerobic strains, and in the Mueller-Hinton culture medium (cation-adjusted) for the aerobic bacteria.

The tested compounds were dissolved in DMSO, and were diluted as far as 2560 µg/mL with the different media according to the specific requirements for each group of strains. The 96-well sealed micro titter plates containing bacteria were incubated in different laboratory conditions depending on the nature of the microorganism. Thus, the aerobic bacteria were incubated during 16-24 h at 35°C and the so-called fastidious bacteria, such as M. *catarrhalis* and *S*. *pneumoniae,* during 20-24h at 35°C in a microaerobiotic atmosphere containing 5% CO₂ (Anaerocult C, MERCK). The results of these tests are given in Table 1.

**Table 1**

| | Microorganism | | | | | | | | |
|---|---|---|---|---|---|---|---|---|---|
| Compound | (1) | (2) | (3) | (4) | (5) | (6) | (7) | (8) | (9) |
| Ex. 3 | 1 | 1 | 0.5 | 16 | 4 | 4 | 1 | 1 | 8 |
| Ex. 9 | 1 | 0.25 | 1 | 8 | 1 | 2 | 0.5 | 1 | 4 |
| Ex. 11 | 0.5 | 0.125 | 0.25 | 2 | NT (*) | 4 | 0.5 | 0.5 | 8 |
| Ex. 15 | 0.5 | 0.125 | 0.25 | 4 | NT | 8 | 0.5 | 2 | 8 |
| Ex. 17 | 1 | 0.25 | 0.5 | 8 | 1 | 1 | 0.5 | 2 | 1 |
| Ex. 18 | 0.5 | 0.06 | 0.25 | 2 | NT | 4 | 0.25 | 0.5 | 8 |
| Ex. 19 | 0.5 | 0.125 | 0.25 | 2 | NT | 4 | 1 | 1 | 64 |
| Ex. 26 | 2 | 0.5 | 1 | 16 | 1 | 1 | 1 | 2 | 2 |
| Linezolid | 1.00 | 1.00 | 0.50 | 16-32 | 128 | 16 | 2 | 2 | 64 |

| | | | | | | | | | |
|---|---|---|---|---|---|---|---|---|---|
| (1) *S. aureus* ATCC25923 MS (2) *S. pneumoniae* ATCC49619 PR (3) *E. faecium* ATCC51559 MDR (4) *S. aureus* LNZ-R 432 (5) *S. haemolyticus* (6) *H. influenzae* ATCC49247 (7) *B. fragilis* sp. fragilis ATCC25285 (8) *M. catarrhalis* HCl-78 (9) *E. faecium* LNZ-R LR-4 (*) NT: Not Tested | | | | | | | | | |

### Example 41: Pharmaceutical compositions

The following illustrate representative pharmaceutical compositions containing a compound of formula (I) or a pharmaceutically acceptable salt thereof for antimicrobial use in humans or animals:

| Tablet 1 | mg/tablet |
|---|---|
| Active ingredient | 100 |
| Lactose | 179 |
| Croscarmellose sodium | 12 |
| Polyvinylpyrrolidone | 6 |
| Magnesium stearate | 3 |

| Tablet 2 | mg/tablet |
|---|---|
| Active ingredient | 50 |
| Lactose | 229 |
| Croscarmellose sodium | 12 |
| Polyvinylpyrrolidone | 6 |
| Magnesium stearate | 3 |

| Tablet 3 | mg/tablet |
|---|---|
| Active ingredient | 1 |
| Lactose | 92 |
| Croscarmellose sodium | 4 |
| Polyvinylpyrrolidone | 2 |
| Magnesium stearate | 1 |

| Capsule | mg/capsule |
|---|---|
| Active ingredient | 10 |
| Lactose | 389 |
| Croscarmellose sodium | 100 |
| Magnesium stearate | 1 |
| | |

| Injection | 50 mg/mL |
|---|---|
| Active ingredient | 5.0% w/v |
| Isotonic aqueous solution | to 100% |

Buffers, pharmaceutically acceptable co-solvents such as polyethylene glycol, polypropylene glycol, glycerol or ethanol or complexing agents, may be used to aid formulation.

The above formulations may be prepared by well-known conventional procedures in the pharmaceutical art. The tablets 1-3 may be enteric coated by conventional means, for example to provide a coating of cellulose acetate phthalate.

## Claims

1. A compound of formula (I), 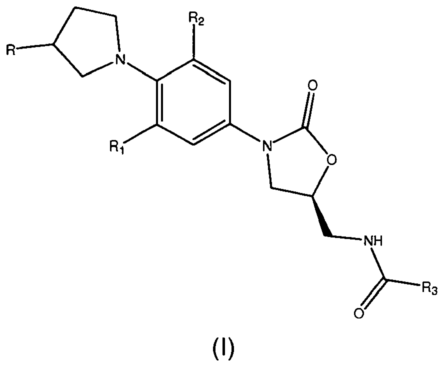 in free or pharmaceutically acceptable salt, solvate, hydrate, or enantiomeric form, wherein:
R is a N-linked 5-membered fully or partially unsaturated heterocyclic ring, containing 0 to 3 further nitrogen heteroatoms,
which is optionally substituted on any available carbon atom with a substituent selected from linear or branched (1-6C)alkyl, (3-6C)cycloalkyl, halogen, OR₄, S(O)ₘR₅, COOH, COOR₆, COR₇, CONH₂, CONHR₈, CONR₉R₁₀, SO₂NH₂, SO₂NHR₁₁, SO₂NR₁₂R₁₃, NH₂, NHR₁₄, NR₁₅R₁₆, NHCOR₁₇, N(R₁₈)COR₁₉, NHSO₂R₂₀, N(R₂₁)SO₂R₂₂, CN, CF₃, NO₂, phenyl optionally substituted with up to three substituents independently selected from linear or branched (1-6C)alkyl, (3-6C)cycloalkyl, halogen, OR₂₃, S(O)ₙR₂₄, NH₂, NHR₂₅, NR₂₆R₂₇, NHCOR₂₈, N(R₂₉)COR₃₀, NHSO₂R₃₁, N(R₃₂)SO₂R₃₃, CN, CF₃, NO₂, and 5-6 membered heteroaryl group containing one to three heteroatoms selected from nitrogen, oxygen and sulfur, optionally substituted with up to three substituents independently selected from linear or branched (1-6C)alkyl, (3-6C)cycloalkyl, halogen, OR₃₄, S(O)ₚR₃₅, NH₂, NHR₃₆, NR₃₇R₃₈, NHCOR₃₉, N(R₄₀)COR₄₁, NHSO₂R₄₂, N(R₄₃)SO₂R₄₄, CN, CF₃, and NO₂,
said ring being optionally fused with a phenyl or 5-6 membered fully or partially unsaturated heterocycle containing one to three heteroatoms selected from nitrogen, oxygen and sulfur to form a benzo-fused or hetero-fused system, wherein the benzo- or hetero- moiety is optionally substituted with up to three substituents independently selected from linear or branched (1-6C)alkyl, (3-6C)cycloalkyl, halogen, OR₄₅, S(O)_{q}R₄₆, NH₂, NHR₄₇, NR₄₈R₄₉, NHCOR₅₀, N(R₅₁)COR₅₂, NHSO₂R₅₃, N(R₅₄)SO₂R₅₅, CN, CF₃, and NO₂;
R₁ and R₂ are radicals identical or different and are independently selected from hydrogen, and fluorine;
R₃ is a linear or branched (1-6C)alkyl group optionally substituted by a group selected from fluorine, hydroxy, and OR₅₆;
R₄ to R₅₆ are identical or different linear or branched (1-6C)alkyl groups; or R₉+R₁₀, R₁₂+R₁₃, R₁₅+R₁₆, R₂₆+R₂₇, R₃₇+R₃₈, and R₄₈+R₄₉ together with the nitrogen atom carrying them, form a monocyclic 5-, 6- or 7-membered saturated heterocycle optionally containing in the cyclic system a second hetero atom selected from oxygen and nitrogen; and
m, n, p and q are identical or different integers independently selected from 0, 1, and 2.

2. The compound according to claim 1, wherein R is selected from the group consisting of benzotriazolyl, 1-imidazolyl, 4-acetylpyrazol-1-yl, 4-bromopyrazol-1-yl, 4-nitropyrazolyl, 3-trifluoromethyl-pyrazol-1-yl, 3-phenylpyrazol-1-yl, 3-(2-fluorophenyl)-pyrazol-1-yl, 3-(4-trifluoromethylphenyl)-pyrazol-1-yl, 4-(4-fluorophenyl)-pyrazol-1-yl, 4-(2-methoxyphenyl)-pyrazol-1-yl, 4-(4-nitrophenyl)-pyrazol-1-yl, 4-(2-trifluoromethyl-phenyl)-pyrazol-1-yl, 4-pyrazin-2-yl-pyrazol-1-yl, 4-pyridin-4-yl-pyrazol-1-yl, 4-pyrimidin-4-yl-pyrazol-1-yl, 1-tetrazolyl, 2-tetrazolyl, 5-methyltetrazol-2-yl, 5-methylsulfanylltetrazol-2-yl, 5-phenyltetrazol-2-yl, 5-p-tolyltetrazol-2-yl, 5-thiophen-2-yl-tetrazol-2-yl, 1-triazolyl, 2-triazolyl, [1,2,3]triazol-1-yl, [1,2,3]triazol-2-yl, (3-cyanophenyl)-[1,2,3]triazol-2-yl], 4-pyridin-2-yl-[1,2,3]triazol-2-yl, and [1,2,4]triazol-1-yl.

3. The compound according to claim 1, wherein R₁ is fluorine.

4. The compound according to claim 1, wherein R₂ is selected from fluorine and hydrogen.

5. The compound according to claim 1, wherein R₃ is methyl.

6. The compound as claimed in any of the preceding claims, which is selected from the group consisting of:
*N*-((5*S*)-3-{3-fluoro-4-[3-(2-triazolyl)pyrrolidin-1-yl]-phenyl}-2-oxo-5-oxazolidinyl methyl) acetamide;
*N*-((5*S*)-3-{3-fluoro-4-[3-(1-triazolyl)pyrrolidin-1-yl]-phenyl}-2-oxo-5-oxazolidinyl methyl) acetamide;
*N*-((5*S*)-3-[3-fluoro-4-[3-(1-imidazolyl)pyrrolidin-1-yl]-phenyl}-2-oxo-5-oxazolidinyl methyl) acetamide;
*N*-((5*S*)-3-{3-fluoro-4-[3-(2-tetrazolyl)pyrrolidinyl]-phenyl}-2-oxo-5-oxazolidinyl methyl) acetamide;
*N*-((5*S*)-3-{3-fluoro-4-[3-(1-tetrazolyl)pyrrolidinyl]-phenyl}-2-oxo-5-oxazolidinyl methyl) acetamide;
*N*-{(5*S*)-3-[3-fluoro-4-(3-[1, 2, 4]triazol-1-yl-pyrrolidin-1-yl)-phenyl]-2-oxo-5-oxazolidinylmethyl} acetamide;
*N*-((5*S*)-3-{3-fluoro-4-[3-(benzotriazolyl)pyrrolidin-1-yl]-phenyl}-2-oxo-5-oxazolidinyl methyl) acetamide;
*N*-((5*S*)-3-{3-fluoro-4-[3-(4-nitropyrazolyl)pyrrolidinyl]-phenyl}-2-oxo-5-oxazolidinyl methyl) acetamide ;
*N*-((5*S*)-3-{3-fluoro-4-[3-(5-p-tolyltetrazol-2-yl)pyrrolidin-1-yl]-phenyl}-2-oxo-5-oxazolidinylmethyl) acetamide ;
*N*-((5*S*)-3-{3-fluoro-4-[3-(4-pyrimidin-4-yl-pyrazol-1-yl)pyrrolidin-1-yl]-phenyl}-2-oxo-5-oxazolidinylmethyl) acetamide;
*N*-((5*S*)-3-{3-fluoro-4-[3-(4-pyrazin-2-yl-pyrazol-1-yl)pyrrolidin-1-yl]-phenyl}-2-oxo-5-oxazolidinylmethyl) acetamide;
*N*-((5*S*)-3-{3-fluoro-4-[3-(5-phenyltetrazol-2-yl)pyrrolidin-1-yl]-phenyl}-2-oxo-5-oxazolidinylmethyl) acetamide;
*N*-((5*S*)-3-{3-fluoro-4-[3-(5-methylsulfanylltetrazol-2-yl)pyrrolidin-1-yl]-phenyl}-2-oxo-5-oxazolidinylmethyl) acetamide;
*N*-((5*S*)-3-{3-fluoro-4-[3-(5-thiophen-2-yl-tetrazol-2-yl)pyrrolidin-1-yl]-phenyl}-2-oxo-5-oxazolidinylmethyl) acetamide;
*N*-((5*S*)-3-{3-fluoro-4-[3-(5-methyltetrazol-2-yl)pyrrolidin-1-yl]-phenyl}-2-oxo-5-oxazolidinylmethyl) acetamide;
*N*-((5*S*)-3-{3-fluoro-4-[3-(4-bromopyrazol-1-yl)pyrrolidin-1-yl]-phenyl}-2-oxo-5-oxazolidinylmethyl) acetamide;
*N*-((5*S*)-3-{3-fluoro-4-[3-(4-pyridin-4-yl-pyrazol-1-yl)pyrrolidin-1-yl]-phenyl}-2-oxo-5-oxazolidinylmethyl) acetamide;
*N*-[(5*S*)-3-(3-fluoro-4-{3-[4-(4-nitrophenyl)-pyrazol-1-yl]pyrrolidin-1-yl}-phenyl)-2-oxo-5-oxazolidinylmethyl] acetamide;
*N*-[(5*S*)-3-(3-fluoro-4-{3-[4-(2-trifluoromethyl-phenyl)-pyrazol-1-yl]pyrrolidin-1-yl}-phenyl)-2-oxo-5-oxazolidinylmethyl] acetamide;
*N*-[(5*S*)-3-(3-fluoro-4-{3-[4-(2-methoxyphenyl)-pyrazol-1-yl]pyrrolidin-1-yl}-phenyl)-2-oxo-5-oxazolidinylmethyl] acetamide;
*N*-((5*S*)-3-{3-fluoro-4-[3-(4-acetylpyrazol-1-yl)pyrrolidin-1-yl]-phenyl}-2-oxo-5-oxazolidinylmethyl) acetamide;
*N*-((5*S*)-3-{3-fluoro-4-[3-(3-phenylpyrazol-1-yl)pyrrolidin-1-yl]-phenyl}-2-oxo-5-oxazolidinylmethyl) acetamide;
*N*-[(5*S*)-3-(3-fluoro-4-{3-[4-(4-fluorophenyl)-pyrazol-1-yl]pyrrolidin-1-yl}-phenyl)-2-oxo-5-oxazolidinylmethyl] acetamide;
*N*-[(5*S*)-3-(3-fluoro-4-{3-[3-(2-fluorophenyl)-pyrazol-1-yl]pyrrolidin-1-yl}-phenyl)-2-oxo-5-oxazolidinylmethyl] acetamide;
*N*-((5*S*)-3-{3-fluoro-4-[3-(3-trifluoromethyl-pyrazol-1-yl)pyrrolidin-1-yl]-phenyl}-2-oxo-5-oxazolidinylmethyl) acetamide;
*N*-[(5*S*)-3-(3-fluoro-4-{3-[3-(4-trifluoromethyl-phenyl)-pyrazol-1-yl]pyrrolidin-1-yl}-phenyl)-2-oxo-5-oxazolidinylmethyl] acetamide;
*N*-((5*S*)-3-{3-fluoro-4-[3-(4-pyridin-2-yl-[1,2,3]triazol-2-yl)pyrrolidin-1-yl]-phenyl}-2-oxo-5-oxazolidinylmethyl) acetamide;
*N*-[(5*S*)-3-(3-fluoro-4-{3-[4-(3-cyanophenyl)-[1,2,3]triazol-2-yl]pyrrolidin-1-yl}-phenyl)-2-oxo-5-oxazolidinylmethyl] acetamide;
*N*-{(5*S*)-3-[3, 5-difluoro-4-[3-([1,2,3]triazol-2-yl pyrrolidin-1-yl) phenyl]-2-oxo-5-oxazolidinyl methyl} acetamide;
*N*-{(5*S*)-3-[3, 5-difluoro-4-[3-([1,2,3]triazol-1-yl pyrrolidin-1-yl) phenyl]-2-oxo-5-oxazolidinyl methyl} acetamide;
*N*-{(5*S*)-3-[3, 5-difluoro-4-[3-(tetrazol-2-yl pyrrolidin-1-yl) phenyl]-2-oxo-5-oxazolidinyl methyl} acetamide;
*N*-{(5*S*)-3-[3, 5-difluoro-4-[3-(tetrazol-1-yl pyrrolidin-1-yl) phenyl]-2-oxo-5-oxazolidinyl methyl} acetamide ;
*N*-((5*S*)-3-{3-fluoro-4-[3(*R*)-(2-triazolyl)pyrrolidin-1-yl]-phenyl}-2-oxo-5-oxazolidinyl methyl) acetamide;
*N*-((5*S*)-3-{3-fluoro-4-[3(*R*)-(1-triazolyl)pyrrolidin-1-yl]-phenyl}-2-oxo-5-oxazolidinyl methyl) acetamide;
*N*-((5*S*)-3-[3-fluoro-4-[3(*R*)-(1-imidazolyl)pyrrolidin-1-yl]-phenyl}-2-oxo-5-oxazolidinyl methyl) acetamide;
*N*-((5*S*)-3-{3-fluoro-4-[3(*R*)-(2-tetrazolyl)pyrrolidinyl]-phenyl}-2-oxo-5-oxazolidinyl methyl) acetamide;
*N*-((5*S*)-3-{3-fluoro-4-[3(*R*)-(1-tetrazolyl)pyrrolidinyl]-phenyl}-2-oxo-5-oxazolidinyl methyl) acetamide;
*N*{(5*S*)-3-[3-fluoro-4-(3(*R*)-[1, 2, 4]triazol-1-yl-pyrrolidin-1-yl)-phenyl]-2-oxo-5-oxazolidinylmethyl} acetamide;
*N*-((5*S*)-3-{3-fluoro-4-[3(R)-(benzotriazolyl)pyrrolidin-1-yl]-phenyl}-2-oxo-5-oxazolidinyl methyl) acetamide;
*N*-((5*S*)-3-{3-fluoro-4-[3(R)-(4-nitropyrazolyl)pyrrolidinyl]-phenyl}-2-oxo-5-oxazolidinyl methyl) acetamide ;
*N*-((5*S*)-3-{3-fluoro-4-[3(*R*)-(5-*p*-tolyltetrazol-2-yl)pyrrolidin-1-yl]-phenyl}-2-oxo-5-oxazolidinylmethyl) acetamide ;
*N*-((5*S*)-3-{3-fluoro-4-[3(R)-(4-pyrimidin-4-yl-pyrazol-1-yl)pyrrolidin-1-yl]-phenyl}-2-oxo-5-oxazolidinylmethyl) acetamide;
*N*-((5*S*)-3-{3-fluoro-4-[3(*R*)-(4-pyrazin-2-yl-pyrazol-1-yl)pyrrolidin-1-yl]-phenyl}-2-oxo-5-oxazolidinylmethyl) acetamide;
*N*-((5*S*)-3-{3-fluoro-4-[3(*R*)-(5-phenyltetrazol-2-yl)pyrrolidin-1-yl]-phenyl}-2-oxo-5-oxazolidinylmethyl) acetamide;
*N*-((5*S*)-3-{3-fluoro-4-[3(*R*)-(5-methylsulfanylltetrazol-2-yl)pyrrolidin-1-yl]-phenyl}-2-oxo-5-oxazolidinylmethyl) acetamide;
*N*-((5*S*)-3-{3-fluoro-4-[3(*R*)-(5-thiophen-2-yl-tetrazol-2-yl)pyrrolidin-1-yl]-phenyl}-2-oxo-5-oxazolidinylmethyl) acetamide;
*N*-((5*S*)-3-{3-fluoro-4-[3(*R*)-(5-methyltetrazol-2-yl)pyrrolidin-1-yl]-phenyl}-2-oxo-5-oxazolidinylmethyl) acetamide;
*N*-((5*S*)-3-{3-fluoro-4-[3(*R*)-(4-bromopyrazol-1-yl)pyrrolidin-1-yl]-phenyl}-2-oxo-5-oxazolidinylmethyl) acetamide;
*N*-((5*S*)-3-{3-fluoro-4-[3(*R*)-(4-pyridin-4-yl-pyrazol-1-yl)pyrrolidin-1-yl]-phenyl}-2-oxo-5-oxazolidinylmethyl) acetamide;
*N*-[(5*S*)-3-(3-fluoro-4-{3(*R*)-[4-(4-nitrophenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*R*)-[4-(2-trifluoromethyl-phenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*R*)-[4-(2-methoxyphenyl)-pyrazol-1-yl]pyrrolidin-1-yl}-phenyl)-2-oxo-5-oxazolidinylmethyl] acetamide;
*N*-((5*S*)-3-{3-fluoro-4-[3(*R*)-(4-acetylpyrazol-1-yl)pyrrolidin-1-yl]-phenyl}-2-oxo-5-oxazolidinylmethyl) acetamide;
*N*-((5*S*)-3-{3-fluoro-4-[3(*R*)-(3-phenylpyrazol-1-yl)pyrrolidin-1-yl]-phenyl}-2-oxo-5-oxazolidinylmethyl) acetamide;
*N*-[(5*S*)-3-(3-fluoro-4-(3(*R*-[4-(4-fluorophenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*R*)-[3-(2-fluorophenyl)-pyrazol-1-yl]pyrrolidin-1-yl}-phenyl)-2-oxo-5-oxazolidinylmethyl] acetamide;
*N*-((5*S*)-3-{3-fluoro-4-[3(*R*)-(3-trifluoromethyl-pyrazol-1-yl)pyrrolidin-1-yl]-phenyl}-2-oxo-5-oxazolidinylmethyl) acetamide;
*N*-[(5*S*)-3-(3-fluoro-4-{3(*R*)-[3-(4-trifluoromethyl-phenyl)-pyrazol-1-yl]pyrrolidin-1-yl}-phenyl)-2-oxo-5-oxazolidinylmethyl] acetamide;
*N*-((5*S*)-3-{3-fluoro-4-[3(*R*)-(4-pyridin-2-yl-[1,2,3]triazol-2-yl)pyrrolidin-1-yl]-phenyl}-2-oxo-5-oxazolidinylmethyl) acetamide;
*N*-[(5*S*)-3-(3-fluoro-4-{3(*R*)-[4-(3-cyanophenyl)-[1,2,3]triazol-2-yl]pyrrolidin-1-yl}-phenyl)-2-oxo-5-oxazolidinylmethyl] acetamide;
*N*-{(5*S*)-3-[3, 5-difluoro-4-[3(*R*)-([1,2,3]triazol-2-yl pyrrolidin-1-yl) phenyl]-2-oxo-5-oxazolidinyl methyl} acetamide;
*N*-{(5*S*)-3-[3, 5-difluoro-4-[3(*R*)-([1,2,3]triazol-1-yl pyrrolidin-1-yl) phenyl]-2-oxo-5-oxazolidinyl methyl} acetamide;
*N*-{(5*S*)-3-[3, 5-difluoro-4-[3(*R*)-(tetrazol-2-yl pyrrolidin-1-yl) phenyl]-2-oxo-5-oxazolidinyl methyl} acetamide;
*N*-{(5*S*)-3-[3, 5-difluoro-4-[3(*R*)-(tetrazol-1-yl pyrrolidin-1-yl) phenyl]-2-oxo-5-oxazolidinyl methyl} acetamide ;
*N*-((5*S*)-3-{3-fluoro-4-[3(*S*)-(2-triazolyl)pyrrolidin-1-yl]-phenyl}-2-oxo-5-oxazolidinyl methyl) acetamide;
*N*-((5*S*)-3-{3-fluoro-4-[3(*S*)-(1-triazolyl)pyrrolidin-1-yl]-phenyl}-2-oxo-5-oxazolidinyl methyl) acetamide;
*N*-((5*S*)-3-[3-fluoro-4-[3(*S*)-(1-imidazolyl)pyrrolidin-1-yl]-phenyl}-2-oxo-5-oxazolidinyl methyl) acetamide;
*N*-((5*S*)-3-(3-fluoro-4-[3(*S*)-(2-tetrazolyl)pyrrolidinyl]-phenyl}-2-oxo-5-oxazolidinyl methyl) acetamide;
*N*-((5*S*)-3-{3-fluoro-4-[3(*S*)-(1-tetrazolyl)pyrrolidinyl]-phenyl}-2-oxo-5-oxazolidinyl methyl) acetamide;
*N*-{(5*S*)-3-[3-fluoro-4-(3(*S*)-[1, 2, 4]triazol-1-yl-pyrrolidin-1-yl)-phenyl]-2-oxo-5-oxazolidinylmethyl} acetamide;
*N*-((5*S*)-3-{3-fluoro-4-[3(*S*)-(benzotriazolyl)pyrrolidin-1-yl]-phenyl}-2-oxo-5-oxazolidinyl methyl) acetamide;
*N*-((5S)-3-{3-fluoro-4-[3(*S*)-(4-nitropyrazolyl)pyrrolidinyl]-phenyl}-2-oxo-5-oxazolidinyl methyl) acetamide;
*N*-((5*S*)-3-{3-fluoro-4-[3(*S*)-(5-p-tolyltetrazol-2-yl)pyrrolidin-1-yl]-phenyl}-2-oxo-5-oxazolidinylmethyl) acetamide;
*N*-((5*S*)-3-{3-fluoro-4-[3(*S*)-(4-pyrimidin-4-yl-pyrazol-1-yl)pyrrolidin-1-yl]-phenyl}-2-oxo-5-oxazolidinylmethyl) acetamide;
*N*-((5*S*)-3-{3-fluoro-4-[3(*S*)-(4-pyrazin-2-yl-pyrazol-1-yl)pyrrolidin-1-yl]-phenyl}-2-oxo-5-oxazolidinylmethyl) acetamide;
*N*-((5*S*)-3-{3-fluoro-4-[3(*S*)-(5-phenyltetrazol-2-yl)pyrrolidin-1-yl]-phenyl}-2-oxo-5-oxazolidinylmethyl) acetamide;
*N*-((5*S*)-3-{3-fluoro-4-[3(*S*)-(5-methylsulfanylltetrazol-2-yl)pyrrolidin-1-yl]-phenyl}-2-oxo-5-oxazolidinylmethyl) acetamide;
*N*-((5*S*)-3-{3-fluoro-4-[3(*S*)-(5-thiophen-2-yl-tetrazol-2-yl)pyrrolidin-1-yl]-phenyl}-2-oxo-5-oxazolidinylmethyl) acetamide;
*N*-((5*S*)-3-{3-fluoro-4-[3(*S*)-(5-methyltetrazol-2-yl)pyrrolidin-1-yl]-phenyl}-2-oxo-5-oxazolidinylmethyl) acetamide;
*N*-((5*S*)-3-{3-fluoro-4-[3(*S*)-(4-bromopyrazol-1-yl)pyrrolidin-1-yl]-phenyl}-2-oxo-5-oxazolidinylmethyl) acetamide;
*N*-((5*S*)-3-{3-fluoro-4-[3(*S*)-(4-pyridin-4-yl-pyrazol-1-yl)pyrrolidin-1-yl]-phenyl}-2-oxo-5-oxazolidinylmethyl) acetamide;
*N*-[(5*S*)-3-(3-fluoro-4-{3(*S*)-[4-(4-nitrophenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*S*)-[4-(2-trifluoromethyl-phenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*S*)-[4-(2-methoxyphenyl)-pyrazol-1-yl]pyrrolidin-1-yl}-phenyl)-2-oxo-5-oxazolidinylmethyl] acetamide;
*N*-((5*S*)-3-{3-fluoro-4-[3(*S*)-(4-acetylpyrazol-1-yl)pyrrolidin-1-yl]-phenyl}-2-oxo-5-oxazolidinylmethyl) acetamide;
*N*-((5*S*)-3-{3-fluoro-4-[3(*S*)-(3-phenylpyrazol-1-yl)pyrrolidin-1-yl]-phenyl}-2-oxo-5-oxazolidinylmethyl) acetamide;
*N*-[(5*S*)-3-(3-fluoro-4-{3(*S*)-[4-(4-fluorophenyl)-pyrazol-1-yl]pyrrolidin-1-yl}-phenyl)-2-oxo-5-oxazolidinylmethyl] acetamide;
*N*-[(5*S*)-3-(3-fluoro-4-{3(*S*)-[3-(2-fluorophenyl)-pyrazol-1-yl]pyrrolidin-1-yl}-phenyl)-2-oxo-5-oxazolidinylmethyl] acetamide;
*N*-((5*S*)-3-{3-fluoro-4-[3(*S*)-(3-trifluoromethyl-pyrazol-1-yl)pyrrolidin-1-yl]-phenyl}-2-oxo-5-oxazolidinylmethyl) acetamide;
*N*-[(5*S*)-3-(3-fluoro-4-{3(*S*)-[3-(4-trifluoromethyl-phenyl)-pyrazol-1-yl]pyrrolidin-1-yl}-phenyl)-2-oxo-5-oxazolidinylmethyl] acetamide;
*N*-((5S)-3-{3-fluoro-4-[3(*S*)-(4-pyridin-2-yl-[1,2,3]triazol-2-yl)pyrrolidin-1-yl]-phenyl}-2-oxo-5-oxazolidinylmethyl) acetamide;
*N*-[(5S)-3-(3-fluoro-4-{3(*S*)-[4-(3-cyanophenyl)-[1,2,3]triazol-2-yl]pyrrolidin-1-yl}-phenyl)-2-oxo-5-oxazolidinylmethyl] acetamide;
*N*-{(5S)-3-[3, 5-difluoro-4-[3(*S*)-([1,2,3]triazol-2-yl pyrrolidin-1-yl) phenyl]-2-oxo-5-oxazolidinyl methyl} acetamide;
*N*-{(5S)-3-[3, 5-difluoro-4-[3(*S*)-([1,2,3]triazol-1-yl pyrrolidin-1-yl) phenyl]-2-oxo-5-oxazolidinyl methyl} acetamide;
*N*-{(5S)-3-[3, 5-difluoro-4-[3(*S*)-(tetrazol-2-yl pyrrolidin-1-yl) phenyl]-2-oxo-5-oxazolidinyl methyl} acetamide; and
*N*-{(5S)-3-[3, 5-difluoro-4-[3(*S*)-(tetrazol-1-yl pyrrolidin-1-yl) phenyl]-2-oxo-5-oxazolidinyl methyl} acetamide.

7. A process for preparing a compound of formula (I) in free or pharmaceutically acceptable salt, solvate, hydrate, or enantiomeric form that comprises:
(i) a) reacting an intermediate of formula (II), 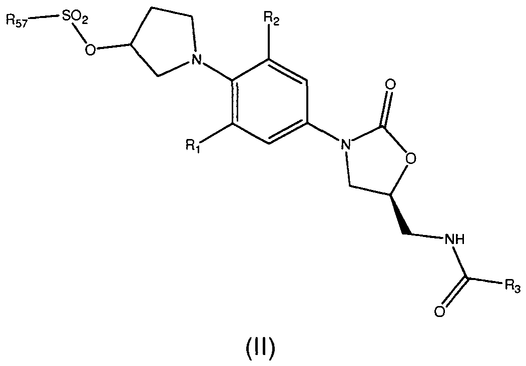 wherein R₁, R₂ and R₃ are as defined above, and R₅₇ is selected from methyl, phenyl, *p*-tolyl, *p*-bromophenyl, p-nitrophenyl, trifluoromethyl, and 2,2,2-trifluoroethyl, with an intermediate of formula RH (III), wherein R is as defined above; or
b) reacting an intermediate of formula (IV), 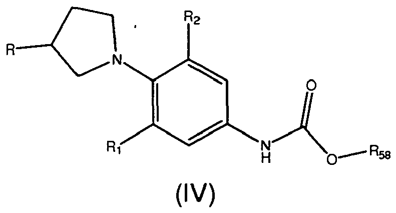 wherein R, R₁ and R₂ are as defined above and R₅₈ is selected from linear or branched (1-6C)alkyl, and benzyl optionally substituted in the phenyl ring by up to three linear or branched (1-6C)alkyl groups, with an intermediate of formula (V), 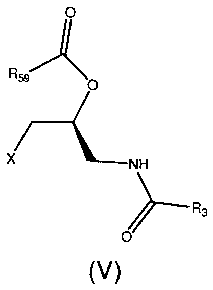 wherein R₃ is as defined above, R₅₉ is a linear or branched (1-6C)alkyl group, and X is a halogen atom; and
(ii) recovering the resultant compound of formula (I) in free or pharmaceutically acceptable salt, solvate, hydrate, or enantiomeric form.

8. The process of claim 7 wherein R₅₇ is methyl, R₅₈ is benzyl, R₅₉ is methyl, and X is bromine.

9. A pharmaceutical composition comprising a therapeutically effective amount of the compound of formula (I) as defined in any of the claims 1 to 6, together with the appropriate amounts of pharmaceutical excipients or carriers.

10. A compound of formula (I) as defined in any of the claims 1 to 6 for use as a medicament.

11. A compound of formula (I) as defined in any of the claims 1 to 6 for use in the treatment of bacterial infections.

## Patentansprüche

1. Verbindung der Formel (I) 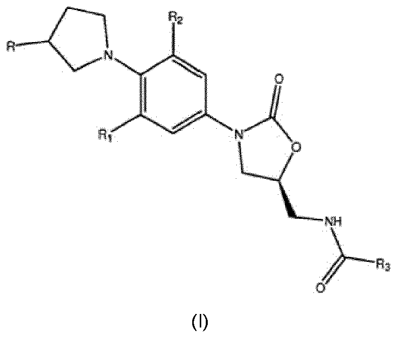 in freier Form oder in Form eines pharmazeutisch akzeptablen Salzes, Solvates, Hydrates davon oder als Enantiomer davon, worin:
R ein mit einem Stickstoffatom gebundener 5-gliedriger völlig oder teils ungesättigter heterozyklischer Ring ist, der 0 bis 3 weitere Stickstoffheteroatome enthält,
der gegebenenfalls substituiert ist an irgendeinem verfügbarem Kohlenstoffatom mit einem Substituenten, der ausgewählt ist aus linearer oder verzweigter (1-6C)-Alkylgruppe, (3-6C)-Cycloalkyl, Halogen, OR₄, S(O)ₘR₅, COOH, COOR₆, COR₇, CONH₂, CONHR₈, CONR₉R₁₀, SO₂NH₂, SO₂NHR₁₁, SO₂NR₁₂R₁₃, NH₂, NHR₁₄, NR₁₅R₁₆, NHCOR₁₇, N(R₁₈)COR₁₉, NHSO₂R₂₀, N(R₂₁)SO₂R₂₂, CN, CF₃, NO₂, Phenyl gegebenenfalls substituiert mit bis zu drei Substituenten, die unabhängig voneinander ausgewählt sind aus linearer oder verzweigter (1-6C)-Alkylgruppe, (3-6C)-Cycloalkyl, Halogen, OR₂₃, S(O)ₙR₂₄, NH₂, NHR₂₅, NR₂₆R₂₇, NHCOR₂₈, N(R₂₉)COR₃₀, NHSO₂R₃₁, N(R₃₂)SO₂R₃₃, CN, CF₃, NO₂, und 5- bis 6-gliedriger Heteroarylgruppe, die ein bis drei Heteroatome enthält, die ausgewählt sind aus Stickstoff, Sauerstoff und Sulfur,
gegebenenfalls substituiert mit bis zu drei Substituenten, die unabhängig voneinander ausgewählt sind aus linearer oder verzweigter (1-6C)-Alkylgruppe, (3-6C)-Cycloalkyl, Halogen, OR₃₄, S(O)ₚR₃₅, NH₂, NHR₃₆, NR₃₇R₃₈, NHCOR₃₉, N(R₄₀)COR₄₁, NHSO₂R₄₂, N(R₄₃)SO₂R₄₄, CN, CF₃, und NO₂,
wobei der Ring gegebenenfalls fusioniert ist mit einem Phenyl oder mit einem 5- bis 6-gliedrigen völlig oder teils ungesättigten Heterozyklus, der ein bis drei Heteroatome enthält, die ausgewählt sind aus Stickstoff, Sauerstoff und Sulfur um ein Benzofusioniert oder Hetero-fusioniert System zu bilden, worin die Benzo- oder Heteroeinheit gegebenenfalls mit bis zu drei Substituenten substituiert ist, die unabhängig voneinander ausgewählt sind aus linearer oder verzweigter (1-6C)-Alkylgruppe, (3-6C)-Cycloalkylgruppe, Halogen, OR₄₅, S(O)_{q}R₄₆, NH₂, NHR₄₇, NR₄₈R₄₉, NHCOR₅₀, N(R₅₁)COR₅₂, NHSO₂R₅₃, N(R₅₄)SO₂R₅₃, CN, CF₃, und NO₂;
R₁ und R₂ sind gleiche oder verschiedene Radikale, die unabhängig voneinander ausgewählt sind aus Hydrogen und Fluor;
R₃ ist eine lineare oder verzweigte (1-6C)-Alkylgruppe gegebenenfalls substituiert mit einer Gruppe ausgewählt aus Fluor, Hydroxygruppe, und OR₅₆;
R₄ bis R₅₆ sind gleiche oder verschiedene lineare oder verzweigte (1-6C)-Alkylgruppen;
oder R₉+R₁₀, R₁₂+R₁₃, R₁₅+R₁₆, R₂₆+R₂₇, R₃₇+R₃₈ und R₄₈+R₄₉ bilden zusammen mit dem an ihnen gebundenen Stickstoffatom einen monozyklischen 5-, 6- oder 7-gliedrigen gesättigten Heterozyklus, der gegebenenfalls im zyklischen System ein zweites Heteroatom enthält, das ausgewählt ist aus Sauerstoff oder Stickstoff; und m, n, p und q gleiche oder verschiedene Ganzzahlen sind, die unabhängig voneinander ausgewählt sind aus 0, 1, und 2.

2. Verbindung nach Anspruch 1, worin R ausgewählt ist aus der Gruppe bestehend aus: Benzotriazolyl, 1-Imidazolyl, 4-Acetylpyrazol-1-yl, 4-Bromopyrazol-1-yl, 4-Nitropyrazolyl, 3-Trifluoromethyl-pyrazol-1-yl, 3-Phenylpyrazol-1-yl, 3-(2-Fluorophenyl)-pyrazol-1-yl, 3-(4-Trifluoromethyl-phenyl)-pyrazol-1-yl, 4-(4-Fluorophenyl)-pyrazol-1-yl, 4-(2-Methoxyphenyl)-pyrazol-1-yl, 4-(4-Nitrophenyl)-pyrazol-1-yl, 4-(2-Trifluoromethyl-phenyl)-pyrazol-1-yl, 4-Pyrazin-2-yl-pyrazol-1-yl, 4-Pyridin-4-yl-pyrazol-1-yl, 4-Pyrimidin-4-yl-pyrazol-1-yl, 1-Tetrazolyl, 2-Tetrazolyl, 5-Methyltetrazol-2-yl, 5-Methylsulfanyltetrazol-2-yl, 5-Phenyltetrazol-2-yl, 5-p-Tolyltetrazol-2-yl, 5-Thiophen-2-yl-tetrazol-2-yl, 1-Triazolyl, 2-Triazolyl, [1,2,3]Triazol-1-yl, [1,2,3]Triazol-2-yl, (3-Cyanophenyl)-[1,2,3]triazol-2-yl], 4-Pyridin-2-yl-[1,2,3]triazol-2-yl, und [1,2,4]Triazol-1-yl.

3. Verbindung nach Anspruch 1, worin R₁ Fluor ist.

4. Verbindung nach Anspruch 1, worin R₂ ausgewählt aus Fluor oder Hydrogen ist.

5. Verbindung nach Anspruch 1, worin R₃ Methyl ist.

6. Verbindung nach einem der vorangehenden Ansprüche, die ausgewählt ist aus der Gruppe bestehend aus:
N-((5S)-3-{3-Fluoro-4-[3-(2-triazolyl)pyrrolidin-1-yl]-phenyl}-2-oxo-5-oxazolidinyl-methyl)acetamid;
N-((5S)-3-{3-Fluoro-4-[3-(1-triazolyl)pyrrolidin-1-yl]-phenyl}-2-oxo-5-oxazolidinyl-methyl)acetamid;
N-((5S)-3-[3-Fluoro-4-[3-(1-imidazolyl)pyrrolidin-1-yl]-phenyl}-2-oxo-5-oxazolidinyl-methyl)acetamid;
N-((5S)-3-{3-Fluoro-4-[3-(2-tetrazolyl)pyrrolidinyl]-phenyl}-2-oxo-5-oxazolidinyl-methyl)acetamid;
N-((5S)-3-{3-Fluoro-4-[3-(1-tetrazolyl)pyrrolidinyl]-phenyl}-2-oxo-5-oxazolidinyl-methyl)acetamid;
N-{(5S)-3-[3-Fluoro-4-(3-[1,2,4]triazol-1-yl-pyrrolidin-1-yl)-phenyl]-2-oxo-5-oxazolidinyl-methyl}acetamid;
N-((5S)-3-{3-Fluoro-4-[3-(benzotriazolyl)pyrrolidin-1-yl]-phenyl}-2-oxo-5-oxazolidinyl-methyl)acetamid;
N-((5S)-3-{3-Fluoro-4-[3-(4-nitropyrazolyl)pyrrolidinyl]-phenyl}-2-oxo-5-oxazolidinyl-methyl)acetamid;
N-((5S)-3-{3-Fluoro-4-[3-(5-*p*-tolyltetrazol-2-yl)pyrrolidin-1-yl]-phenyl}-2-oxo-5-oxazolidinyl-methyl)acetamid;
N-((5S)-3-{3-Fluoro-4-[3-(4-pyrimidin-4-yl-pyrazol-1-yl)pyrrolidin-1-yl]-phenyl}-2-oxo-5-oxazolidinyl-methyl)acetamid;
N-((5S)-3-{3-Fluoro-4-[3-(4-pyrazin-2-yl-pyrazol-1-yl)pyrrolidin-1-yl]-phenyl}-2-oxo-5-oxazolidinyl-methyl)acetamid;
N-((5S)-3-{3-Fluoro-4-[3-(5-phenyltetrazol-2-yl)pyrrolidin-1-yl]-phenyl}-2-oxo-5-oxazolidinyl-methyl)acetamid;
N-((5S)-3-{3-Fluoro-4-[3-(5-methylsulfanyltetrazol-2-yl)pyrrolidin-1-yl]-phenyl}-2-oxo-5-oxazolidinyl-methyl)acetamid;
N-((5S)-3-{3-Fluoro-4-[3-(5-thiophen-2-yl-tetrazol-2-yl)pyrrolidin-1-yl]-phenyl}-2-oxo-5-oxazolidinyl-methyl)acetamid;
N-((5S)-3-{3-Fluoro-4-[3-(5-methyltetrazol-2-yl)pyrrolidin-1-yl]-phenyl}-2-oxo-5-oxazolidinyl-methyl)acetamid;
N-((5S)-3-{3-Fluoro-4-[3-(4-bromopyrazol-1-yl)pyrrolidin-1-yl]-phenyl}-2-oxo-5-oxazolidinyl-methyl)acetamid;
N-((5S)-3-{3-Fluoro-4-[3-(4-pyridin-4-yl-pyrazol-1-yl)pyrrolisdin-1-yl]-phenyl}-2-oxo-5-oxazolidinyl-methyl)acetamid;
N-[(5S)-3-(3-Fluoro-4-{3-[4-(4-nitrophenyl)-pyrazol-1-yl]pyrrolidin-1-yl}-phenyl)-2-oxo-5-oxazolidinyl-methyl]acetamid;
N-[(5S)-3-(3-Fluoro-4-{3-[4-(2-trifluoromethyl-phenyl)-pyrazol-1-yl]pyrrolidin-1-yl}-phenyl)-2-oxo-5-oxazol idi nyl-methyl]acetam id;
N-[(5S)-3-(3-Fluoro-4-{3-[4-(2-methoxyphenyl)-pyrazol-1-yl]pyrrolidin-1-yl}-phenyl)-2-oxo-5-oxazolidinyl-methyl]acetamid;
N-((5S)-3-{3-Fluoro-4-[3-(4-acetylpyrazol-1-yl)pyrrolidin-1-yl]-phenyl}-2-oxo-5-oxazolidinyl-methyl)acetamid;
N-((5S)-3-{3-Fluoro-4-[3-(3-phenylpyrazol-1-yl)pyrrolidin-1-yl]-phenyl}-2-oxo-5-oxazolidinyl-methyl)acetamid;
N-[(5S)-3-(3-Fluoro-4-{3-[4-(4-fluorophenyl)-pyrazol-1-yl]pyrrolidin-1-yl}-phenyl)-2-oxo-5-oxazol idi nyl-methyl]acetam id;
N-[(5S)-3-(3-Fluoro-4-{3-[3-(2-fluorophenyl)-pyrazol-1-yl]pyrrolidin-1-yl}-phenyl)-2-oxo-5-oxazol idi nyl-methyl]acetam id;
N-((5S)-3-{3-Fluoro-4-[3-(3-trifluoromethyl-pyrazol-1-yl)pyrrolidin-1-yl]-phenyl}-2-oxo-5-oxazolidinyl-methyl)acetamid;
N-[(5S)-3-(3-Fluoro-4-{3-[3-{4-trifluoromethyl-phenyl)-pyrazol-1-yl]pyrrolidin-1-yl)-phenyl)-2-oxo-5-oxazol idi nyl-methyl]acetam id;
N-((5S)-3-{3-Fluoro-4-[3-(4-pyridin-2-yl-[1,2,3]triazol-2-yl)pyrrolidin-1-yl]-phenyl}-2-oxo-5-oxazol idi nyl-methyl)acetam id;
N-[(5S)-3-(3-Fluoro-4-{3-[4-(3-cyanophenyl)-[1,2,3]triazol-2-yl]pyrrolidin-1-yl}-phenyl)-2-oxo-5-oxazolidinyl-methyl]acetamid;
N-{{5S)-3-[3,5-Difluoro-4-[3-([1,2,3]triazo1-2-yl-pyrrolidin-1 -yl)-phenyl]-2-oxo-5-oxazolidinyl-methyl}acetamid;
N-{(5S)-3-[3,5-Difluoro-4-[3-([1,2,3]thazol-1-yl-pyrrolidin-1-yl)-phenyl]-2-oxo-5-oxazolidinyl-methyl}acetamid;
N-{(5S)-3-[3,5-Difluoro-4-[3-(tetrazol-2-yl-pyrrolidin-1-yl)-phenyl]-2-oxo-5-oxazolidinyl-methyl}acetamid;
N-{(5S)-3-[3,5-Difluoro-4-[3-(tetrazol-1-yl-pyrrolidin-1-yl)-phenyl]-2-oxo-5-oxazolidinyl-methyl}acetamid;
N-((5S)-3-{3-Fluoro-4-[3(R)-(2-triazolyl)pyrrolidin-1-yl]-phenyl}-2-oxo-5-oxazolidinyl-methyl)acetamid;
N-((5S)-3-{3-Fluoro-4-[3(R)-(1-triazolyl)pyrrolidin-1-yl]-phenyl}-2-oxo-5-oxazolidinyl-methyl)acetamid;
N-((5S)-3-[3-Fluoro-4-[3(R)-(1-imidazolyl)pyrrolidin-1-yl]-phenyl}-2-oxo-5-oxazolidinyl-methyl)acetamid;
N-((5S)-3-{3-Fluoro-4-[3(R)-(2-tetrazolyl)pyrrolidinyl]-phenyl}-2-oxo-5-oxazolidinyl-methyl)acetamid;
N-((5S)-3-{3-Fluoro-4-[3(R)-(1-tetrazolyl)pyrrolidinyl]-phenyl}-2-oxo-5-oxazolidinyl-methyl)acetamid;
N-{(5S)-3-[3-Fluoro-4-(3(R)-[1,2,4]triazol-1-yl-pyrrolidin-1-yl)-phenyl]-2-oxo-5-oxazolidinyl-methyl}acetamid;
N-((5S)-3-{3-Fluoro-4-[3(R)-(benzotriazolyl)pyrrolidin-1-yl]-phenyl}-2-oxo-5-oxazolidinyl-methyl)acetamid;
N-((5S)-3-{3-Fluoro-4-[3(R)-(4-nitropyrazolyl)pyrrolidinyl]-phenyl}-2-oxo-5-oxazolidinyl-methyl)acetamid;
N-((5S)-3-{3-Fluoro-4-[3(R)-(5-p-tolyltetrazol-2-yl)pyrrolidin-1-yl]-phenyl}-2-oxo-5-oxazolidinyl-methyl)acetamid;
N-((5S)-3-{3-Fluoro-4-[3(R)-(4-pyrimidin-4-yl-pyrazol-1-yl)pyrrolidin-1-yl]-phenyl}-2-oxo-5-oxazol idi nyl-methyl)acetam id;
N-((5S)-3-{3-Fluoro-4-[3(R)-(p-pyrazin-2-yl-pyrazol-yl)pyrrolidin-1-yl]-phenyl}-2-oxo-5-oxazolidinyl-methyl)acetamid;
N-((5S)-3-{3-Fluoro-4-[3(R)-(5-phenyltetrazol-2-yl)pyrrolidin-1-yl]-phenyl}-2-oxo-5-oxazolidinyl-methyl)acetamid;
N-((5S)-3-(3-Fluoro-4-[3(R)-(5-methylsulfanyltetrazol-2-yl)pyrrolidin-1-yl]-phenyl}-2-oxo-5-oxazol idi nyl-methyl)acetam id;
N-((5S)-3-{3-Fluoro-4-[3(R)-(5-thiophen-2-yl-tetrazol-2-yl)pyrrolidin-1-yl]-phenyl}-2-oxo-5-oxazol idi nyl-methyl)acetam id;
N-((5S)-3-{3-Fluoro-4-[3(R)-(5-methyltetrazol-2-yl)pyrrolidin-1-yl]-phenyl}-2-oxo-5-oxazolidinyl-methyl)acetamid;
N-((5S)-3-{3-Fluoro-4-[3(R)-(4-bromopyrazol-1-yl)pyrrolidin-1-yl]-phenyl}-2-oxo-5-oxazolidinyl-methyl)acetamid;
N-((5S)-3-{3-Fluoro-4-[3(R)-(4-pyridin-4-yl-pyrazol-1-yl)pyrrolidin-1-yl]-phenyl}-2-oxo-5-oxazolidinyl-methyl)acetamid;
N-[(5S)-3-(3-Fluoro-4-{3(R)-[4-(4-nitrophenyl)-pyrazol-1-yl]pyrrolidin-1-yl}-phenyl)-2-oxo-5-oxazolidinyl-methyl]acetamid;
N-[(5S)-3-(3-Fluoro-4-(3(R)-[4-(2-trifluoromethyl-phenyl)-pyrazol-1-yl]pyrrolidin-1-yl}-phenyl)-2-oxo-5-oxazol idi nyl-methyl]acetam id;
N-[(5S)-3-(3-fluoro-4-{3(R)-[4-(2-methoxyphenyl)-pyrazol-1-yl]pyrrolidin-1-yl}-phenyl)-2-oxo-5-oxazolidinyl-methyl]acetamid;
N-((5S)-3-{3-Fluoro-4-[3(R)-{4-acetylpyrazol-1-yl)pyrrolidin-1-yl]-phenyl}-2-oxo-5-oxazolidinyl-methyl)acetamid;
N-((5S)-3-{3-Fluoro-4-[3(R)-(3-phenylpyrazol-1-yl)pyrrolidin-1-yl]-phenyl}-2-oxo-5-oxazolidinyl-methyl)acetamid;
N-[(5S)-3-(3-Fluoro-4-(3(R)-[4-(4-fluorophenyl)-pyrazol-1-yl]pyrrolidin-1-yl}-phenyl)-2-oxo-5-oxazolidinyl-methyl]acetamid;
N-[(5S)-3-(3-Fluoro-4-{3(R)-[3-(2-fluorophenyl)-pyrazol-1-yl]pyrrolidin-1-yl}-phenyl)-2-oxo-5-oxazolidinyl-methyl]acetamid;
N-((5S)-3-{3-Fluoro-4-[3(R)-(3-trifluoromethyl-pyrazol-1-yl)pyrrolidin-1-yl]-phenyl}-2-oxo-5-oxazolidinyl-methyl)acetamid;
N-[(5S)-3-(3-Fluoro-4-{3(R)-[3-(4-trifluoromethyl-phenyl)-pyrazol-1-yl]pyrrolidin-1-yl}-phenyl)-2-oxo-5-oxazol idi nyl-methyl]acetam id;
N-((5S)-3-{3-Fluoro-4-[3(R)-(4-pyridin-2-yl-[1,2,3]tria20l-2-yl)pyrrolidin-1-yl]-phenyl}-2-oxo-5-oxazolidinyl-methyl)acetamid;
N-[(5S)-3-(3-Fluoro-4-{3(R)-[4-(3-cyanophenyl)-[1,2,3]triazol-2-yl]pyrrolidin-1-yl}-phenyl)-2-oxo-5-oxazolidinyl-methyl]acetamid;
N-{(5S)-3-[3,5-Difluoro-4-[3(R)-([1,2,3]triazol-2-yl-pyrrolidin-1-yl)-phenyl]-2-oxo-5-oxazolidinyl-methyl}acetamid;
N(5S)-3-[3,5-Difluoro-4-[3(R)-([1,2,3]triazol-1-yl-pyrrolidin-1-yl)-phenyl]-2-oxo-5-oxazolidinyl-methyl}acetamid;
N-{(5S)-3-[3,5-Difluoro-4-[3(R)-(tetrazol-2-yl-pyrrolidin-1-yl)-phenyl]-2-oxo-5-oxazolidinyl-methyl}acetamid;
N-{(5S)-3-[3,5-Difluoro-4-[3(R)-(tetrazol-1-yl-pyrrolidin-1-yl)-phenyl]-2-oxo-5-oxazolidinyl-methyl}acetamid;
N-((5S)-3-{3-Fluoro-4-[3(S)-(2-triazolyl)pyrrolidin-1-yl]-phenyl}-2-oxo-5-oxazolidinyl-methyl)acetamid;
N-((5S)-3-{3-Fluoro-4-[3(S)-(1-triazolyl)pyrrolidin-1-yl]-phenyl}-2-oxo-5-oxazolidinyl-methyl)acetamid;
N-((5S)-3-[3-Fluoro-4-[3(S)-(1-imidazolyl)pyrrolidin-1-yl]-phenyl}-2-oxo-5-oxazolidinyl-methyl)acetamid;
N-((5S)-3-{3-Fluoro-4-[3(S)-(2-tetrazolyl)pyrrolidinyl]-phenyl}-2-oxo-5-oxazolidinyl-methyl)acetamid;
N-((5S)-3-{3-Fluoro-4-[3(S)-(1-tetrazolyl)pyrrolidinyl]-phenyl}-2-oxo-5-oxazolidinyl-methyl)acetamid;
N-{(5S)-3-[3-Fluoro-4-(3(S)-[1,2,4]triazol-1-yl-pyrrolidin-1-yl)-phenyl]-2-oxo-5-oxazolidinyl-methyl}acetamid;
N-((5S)-3-(3-Fluoro-4-[3(S)-(benzotriazolyl)pyrrolidin-1-yl]-phenyl)-2-oxo-5-oxazolidinyl-methyl)acetamid;
N-((5S)-3-{3-Fluoro-4-[3(S)-(4-nitropyrazolyl)pyrrolidinyl]-phenyl}-2-oxo-5-oxazolidinyl-methyl)acetamid;
N-((5S)-3-{3-Fluoro-4-[3(S)-(5-p-tolyltetrazol-2-yl)pyrrolidin-1-yl]-phenyl}-2-oxo-5-oxazolidinyl-methyl)acetamid;
N-((5S)-3-{3-Fluoro-4-[3(S)-(4-pyrimidin-4-yl-pyrazol-1-yl)pyrrolidin-1-yl]-phenyl}-2-oxo-5-oxazol idi nyl-methyl)acetam id;
N-((5S)-3-{3-Fluoro-4-[3(S)-(4-pyrazin-2-yl-pyrazol-1-yl)pyrrolidin-1-yl]-phenyl}-2-oxo-5-oxazolidinyl-methyl)acetamid;
N-((5S)-3-{3-Fluoro-4-[3(S)-(5-phenyltetrazol-2-yl)pyrrolidin-1-yl]-phenyl}-2-oxo-5-oxazolidinyl-methyl)acetamid;
N-((5S)-3-{3-Fluoro-4-[3(S)-(5-methylsulfanyltetrazol-2-yl)pyrrolidin-1-yl]-phenyl}-2-oxo-5-oxazol idi nyl-methyl)acetam id;
N-((5S)-3-{3-Fluoro-4-[3(S)-(5-thiophen-2-yl-tetrazol-2-yl)pyrrolidin-1-yl]-phenyl}-2-oxo-5-oxazol idi nyl-methyl)acetam id;
N-((5S)-3-{3-Fluoro-4-[3(S)-(5-methyltetrazol-2-yl)pyrrolidin-1-yl]-phenyl}-2-oxo-5-oxazolidinyl-methyl)acetamid;
N-((5S)-3-{3-Fluoro-4-[3(S)-(4-bromopyrazol-1-yl)pyrrolidin-1-yl]-phenyl}-2-oxo-5-oxazolidinyl-methyl)acetamid;
N-((5S)-3-{3-Fluoro-4-[3(S)-(4-pyridin-4-yl-pyrazol-1-yl)pyrrolidin-1-yl]-phenyl}-2-oxo-5-oxazolidinyl-methyl)acetamid;
N-[(5S)-3-(3-Fluoro-4-{3(S)-[4-(4-nitrophenyl)-pyrazol-1-yl]pyrrolidin-1-yl}-phenyl)-2-oxo-5-oxazolidinyl-methyl]acetamid;
N-[(5S)-3-(3-Fluoro-4-{3(S)-[4-(2-trifluoromethyl-phenyl)-pyrazol-1-yl]pyrrolidin-1-yl}-phenyl)-2-oxo-5-oxazolidinyl-methyl]acetamid;
N-[(5S)-3-(3-Fluoro-4-(3(S)-[4-(2-methoxyphenyl)-pyrazol-1-yl]pyrrolidin-1-yl}-phenyl)-2-oxo-5-oxazolidinyl-methyl]acetamid;
N-((5S)-3-{3-Fluoro-4-[3(S)-(4-acetylpyrazol-1-yl)pyrrolidin-1-yl]-phenyl}-2-oxo-5-oxazolidinyl-methyl)acetamid;
N-((5S)-3-{3-Fluoro-4-[3(S)-(3-phenylpyrazol-1-yl)pyrrolidin-1-yl]-phenyl}-2-oxo-5-oxazolidinyl-methyl)acetamid;
N-[(5S)-3-(3-Fluoro-4-{3(S)-[4-(4-fluorophenyl)-pyrazol-1-yl]pyrrolidine-1-yl}-phenyl)-2-oxo-5-oxazolidinyl-methyl]acetamid;
N-[(55)-3-(3-Fluoro-4-(3(S)-[3-(2-fluorophenyl)-pyrazol-1-yl]pyrrolidin-1-yl}-phenyl)-2-oxo-5-oxazolidinyl-methyl]acetamid;
N-((5S)-3-{3-Fluoro-4-[3(S)-(3-trifluoromethyl-pyrazol-1-yl)pyrrolidin-1-yl]-phenyl}-2-oxo-5-oxazolidinyl-methyl)acetamid;
N-[(5S)-3-(3-Fluoro-4-{3(S)-[3-(4-trifluoromethyl-phenyl)-pyrazol-1-yl]pyrrolidin-1-yl}-phenyl)-2-oxo-5-oxazol idi nyl-methyl]acetam id;
N-((5S)-3-{3-Fluoro-4-[3(S)-(4-pyridin-2-yl-[1,2,3]triazol-2-yl)pyrrolidin-1-yl]-phenyl}-2-oxo-5-oxazolidinyl-methyl)acetamid;
N-[(5S)-3-(3-Fluoro-4-{3(S)-[4-(3-cyanophenyl)-[1,2,3]triazol-2-yl]pyrrolidin-1-yl}-phenyl)-2-oxo-5-oxazolidinyl-methyl]acetamid;
N-{(5S)-3-[3,5-Difluoro-4-[3(S)-([1,2,3]triazol-2-yl-pyrrolidin-1-yl)phenyl]-2-oxo-5-oxazolidinyl-methyl}acetamid;
N-{(5S)-3-[3,5-Difluoro-4-[3(S)-([1,2,3]triazol-1-yl-pyrrolidin-1-yl)phenyl]-2-oxo-5-oxazolidinyl-methyl}acetamid;
N-{(5S)-3-[3,5-Difluoro-4-[3(S)-(tetrazol-2-yl-pyrrolidin-1-yl)phenyl]-2-oxo-5-oxazolidinyl-methyl}acetamid; und
N-{(5S)-3-[3,5-Difluoro-4-[3(S)-(tetrazol-1-yl-pyrrolidin-1-yl)phenyl]-2-oxo-5-oxazolidinyl-methyl}acetamid.

7. Verfahren zur Herstellung der Verbindung der Formel (I) in freier Form oder in Form eines pharmazeutisch akzeptablen Salzes, Solvats, Hydrats davon, oder als Enantiomer davon, das folgende Schritte umfasst:
(i) a) Umsetzen eines Zwischenprodukts der Formel (II), 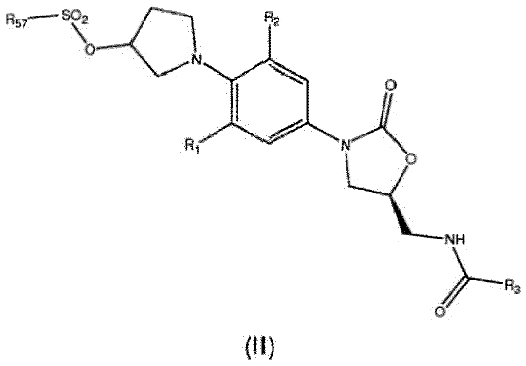 worin R₁, R₂ und R₃ wie oben definiert sind, und R₅₇ ausgewählt ist aus: Methyl, Phenyl, *p*-Tolyl, *p*-Bromophenyl, *p*-Nitrophenyl, Trifluoromethyl und 2,2,2-Trifluoroethyl, mit einem Zwischenprodukt der Formel RH (III), worin R wie oben definiert ist; oder
b) Umsetzen eines Zwischenprodukts der Formel (IV), 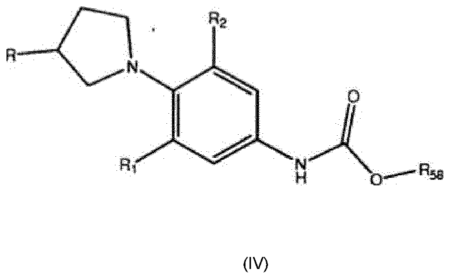 worin R, R₁, und R₂ wie oben definiert sind und R₅₈ ausgewählt ist aus einer linearen oder verzweigten (1-6C)-Alkylgruppe oder Benzyl gegebenenfalls substituiert am Phenylring mit bis zu drei linearen oder verzweigten (1-6C)-Alkylgruppen, mit einem Zwischenprodukt der Formel (V), 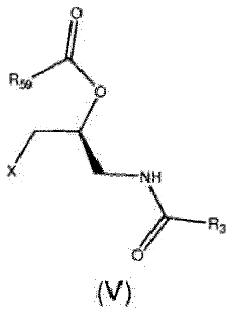 worin R₃ wie oben definiert ist, R₅₉ eine lineare oder verzweigte (1-6C)-Alkyl gruppe ist, und X ein Halogenatom ist; und
(ii) Rückgewinnen der daraus resultierenden Verbindung der Formel (I) in freier Form oder in Form eines pharmazeutisch akzeptablen Salzes, Solvats, Hydrats davon, oder als Enantiomer davon.

8. Verfahren nach Anspruch 7 worin R₅₇ Methyl ist, R₅₈ Benzyl ist, R₅₉ Methyl ist, und X Brom ist.

9. Pharmazeutische Zubereitung, die eine therapeutisch wirksame Menge der Verbindung mit der Formel (I) gemäß der Definition in einem der Ansprüche 1 bis 6 enthält zusammen mit angemessenen Mengen von pharmazeutischen Hilfsstoffen oder Trägersubstanzen.

10. Verbindung der Formel (I) gemäß der Definition in einem der Ansprüche 1 bis 6 zur Verwendung als Medikament.

11. Verbindung der Formel (I) gemäß der Definition in einem der Ansprüche 1 bis 6 zur Verwendung bei der Behandlung bakterieller Infektionen.

## Revendications

1. Composé de formule (I) 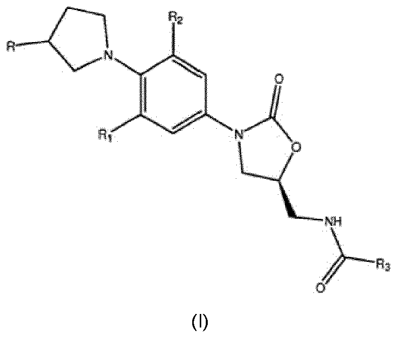 sous forme libre ou de sel, solvate, hydrate ou énantiomère acceptable sur le plan pharmaceutique, dans lequel :
R est un noyau hétérocyclique totalement ou partiellement insaturé à cinq chaînons liés par un azote, contenant de 0 à 3 autres hétéroatomes d'azote,
qui est éventuellement substitué sur n'importe quel atome de carbone disponible par un substituant choisi parmi un groupe alkyle en C1 à C6 linéaire ou ramifié, cycloalkyle en C3 à C6, un halogène, OR₄, S(O)ₘR₅, COOH, COOR₆, COR₇, CONH₂, CONHR₈, CONR₉R₁₀, SO₂NH₂, SO₂NHR₁₁, SO₂NR₁₂R₁₃, NH₂, NHR₁₄, NR₁₅R₁₆, NHCOR₁₇, N(R₁₈)COR₁₉, NHSO₂R₂₀, N(R₂₁)SO₂R₂₂, CN, CF₃, NO₂, un phényle éventuellement substitué par jusqu'à trois substituants indépendamment choisis parmi un groupe alkyle en C1 à C6 linéaire ou ramifié, cycloalkyl en C3 à C6, un halogène, OR₂₃, S(O)ₙR₂₄, NH₂, NHR₂₅, NR₂₆R₂₇, NHCOR₂₈, N(R₂₉)COR₃₀, NHSO₂R₃₁, N(R₃₂)SO₂R₃₃, CN, CF₃, NO₂, et un groupe hétéroaryle à 5 ou 6 chaînons contenant de un à trois hétéroatomes choisis parmi l'azote, l'oxygène et le soufre, éventuellement substitué par jusqu'à trois substituants indépendamment choisis parmi un groupe alkyle en C1 à C6 linéaire ou ramifié, cycloalkyle en C3 à C6, un halogène, OR₃₄, S(O)ₚR₃₅, NH₂, NHR₃₆, NR₃₇R₃₈, NHCOR₃₉, N(R₄₀)COR₄₁, NHSO₂R₄₂, N(R₄₃)SO₂R₄₄, CN, CF₃, et NO₂, étant le noyau éventuellement lié à un phényle ou à un hétérocycle totalement ou
partiellement insaturé à 5 ou 6 chaînons contenant de un a trois hétéroatomes choisis parmi l'azote, l'oxygène et le soufre pour former un système hétérocondensé ou
benzocondensé, dans lequel le fragment benzo ou le fragment hétéro est éventuellement substitué par jusqu'à trois substituants indépendamment choisis parmi un alkyle en C1 à C6 linéaire ou ramifié, cycloalkyle en C3 à C6, un halogène, OR₄₅, S(O)_{q}R₄₆, NH₂, NHR₄₇, NR₄₈R₄₉, NHCOR₅₀, N(R₅₁)COR₅₂, NHSO₂R₅₃, N(R₅₄)SO₂R₅₅, CN, CF₃, et NO₂;
R₁ et R₂ sont des radicaux identiques ou différents et sont indépendamment choisis parmi l'hydrogène et le fluorure ;
R₃ est un groupe alkyle en C1 à C6 linéaire ou ramifié éventuellement substitué par un groupe choisi parmi le fluorure, l'hydroxy et OR₅₆;
R₄ à R₅₆ sont des groupes alkyle en C1 à C6 linéaires ou ramifiés identiques ou
différents; ou R₉+R₁₀, R₁₂+R₁₃, R₁₅+R₁₆, R₂₆+R₂₇, R₃₇+R₃₈ et R₄₈+R₄₉ avec l'atome d'azote qui les porte forment un hétérocycle saturé à cinq, sis ou sept chaînons monocyclique éventuellement contenant dans le système cyclique un deuxième hétéroatome choisi parmi l'oxygène et l'azote ; et
m, n, p et q sont des nombres entiers identiques ou différents indépendamment choisis parmi 0, 1, et 2.

2. Composé selon la revendication 1, dans lequel R est choisi dans le groupe constitué de: benzotriazolyl, 1-imidazolyl, 4-acétylpyrazol-1-yl, 4-bromopyrazol-1-yl, 4-nitropyrazolyl, 3-trifluorométhyl-pyrazol-1-yl, 3-phénylpyrazol-1-yl, 3-(2-fluorophényl)-pyrazol-1-yl, 3-(4-trifluorométhyl-phényl)-pyrazol-1-yl, 4-(4-fluorophényl)-pyrazol-1-yl, 4-(2-méthoxyphényl)-pyrazol-1-yl, 4-(4-nitrophényl)-pyrazol-1-yl, 4-(2-trifluorométhyl-phényl)-pyrazol-1-yl, 4-pyrazin-2-yl-pyrazol-1-yl, 4-pyridin-4-yl-pyrazol-1-yl, 4-pyrimidin-4-yl-pyrazol-1-yl, 1-tetrazolyl, 2-tetrazolyl, 5-méthyltetrazol-2-yl, 5-méthylsulfanyltetrazol-2-yl, 5-phényltetrazol-2-yl, 5-p-tolyltetrazol-2-yl, 5-thiophén-2-yl-tetrazol-2-yl, 1-triazolyl, 2-triazolyl, [1,2,3]triazol-1-yl, [1,2,3]triazol-2-yl, (3-cyanophényl)-[1,2,3]triazol-2-yl], 4-pyridin-2-yl-[1,2,3]triazol-2-yl, et [1,2,4]triazol-1-yl.

3. Composé selon la revendication 1, dans lequel R₁ est le fluorure.

4. Composé selon la revendication 1, dans lequel R₂ est choisi parmi le fluorure et l'hydrogène.

5. Composé selon la revendication 1, dans lequel R₃ est le méthyle.

6. Composé selon l'une quelconque des revendications précédentes choisi dans le groupe constitué de :
N-((5S)-3-{3-fluoro-4-[3-(2-triazolyl)pyrrolidin-1-yl]-phényl}-2-oxo-5-oxazolidinyl-méthyl)acétamide;
N-((5S)-3-{3-fluoro-4-[3-(1-thazolyl)pyrrolidin-1-yl]-phényl}-2-oxo-5-oxazolidinyl-méthyl)acétamide;
N-((5S)-3-[3-fluoro-4-[3-(1-imidazolyl)pyrrolidin-1-yl]-phényl}-2-oxo-5-oxazolidinyl-méthyl)acétamide;
N-((5S)-3-{3-fluoro-4-[3-(2-tetrazolyl)pyrrolidinyl]-phényl}-2-oxo-5-oxazolidinyl-méthyl)acétamide;
N-((5S)-3-{3-fluoro-4-[3-(1-tetrazolyl)pyrrolidinyl]-phényl}-2-oxo-5-oxazolidinyl-méthyl)acétamide;
N-{(5S)-3-[3-fluoro-4-(3-[1,2,4]triazol-1-yl-pyrrolidin-1-yl)-phényl]-2-oxo-5-oxazolidinyl-méthyl}acétamide;
N-((5S)-3-{3-fluoro-4-[3-(benzotriazolyl)pyrrolidin-1-yl]-phényl}-2-oxo-5-oxazolidinyl-méthyl)acétamide;
N-((5S)-3-{3-fluoro-4-[3-(4-nitropyrazolyl)pyrrolidinyl]-phényl}-2-oxo-5-oxazolidinyl-méthyl)acétamide;
N-((5S)-3-{3-fluoro-4-[3-(5-p-tolyltetrazol-2-yl)pyrrolidin-1-yl]-phényl}-2-oxo-5-oxazolidinyl-méthyl)acétamide;
N-((5S)-3-{3-fluoro-4-[3-(4-pyrimidin-4-yl-pyrazol-1-yl)pyrrolidin-1-yl]-phényl}-2-oxo-5-oxazolidinyl-méthyl)acétamide;
N-((5S)-3-{3-fluoro-4-[3-(4-pyrazin-2-yl-pyrazol-1-yl)pyrrolidin-1-yl]-phényl}-2-oxo-5-oxazolidinyl-méthyl)acétamide;
N-((5S)-3-{3-fluoro-4-[3-(5-phényltetrazol-2-yl)pyrrolidin-1-yl]-phényl}-2-oxo-5-oxazolidinyl-méthyl)acétamide;
N-((5S)-3-{3-fluoro-4-[3-(5-méthylsulfanyltetrazol-2-yl)pyrrolidin-1-yl]-phényl}-2-oxo-5-oxazolidinyl-méthyl)acétamide;
N-((5S)-3-{3-fluoro-4-[3-(5-thiophén-2-yl-tetrazol-2-yl)pyrrolidin-1-yl]-phényl}-2-oxo-5-oxazolidinyl-méthyl)acétamide;
N-((5S)-3-{3-fluoro-4-[3-(5-méthyltetrazol-2-yl)pyrrolidin-1-yl]-phényl}-2-oxo-5-oxazolidinyl-méthyl)acétamide;
N-((5S)-3-{3-fluoro-4-[3-(4-bromopyrazol-1-yl)pyrrolidin-1-yl]-phényl}-2-oxo-5-oxazolidinyl-méthyl)acétamide;
N-((5S)-3-(3-fluoro-4-[3-(4-pyridin-4-yl-pyrazol-1-yl)pyrrolidin-1-yl]-phényl}-2-oxo-5-oxazolidinyl-méthyl)acétamide;
N-[(5S)-3-(3-fluoro-4-{3-[4-(4-nitrophényl)-pyrazol-1-yl]pyrrolidin-1-yl}-phényl)-2-oxo-5-oxazolidinyl-méthyl]acétamide;
N-[(5S)-3-(3-fluoro-4-{3-[4-(2-trifluorométhyl-phényl)-pyrazol-1-yl]pyrrolidin-1-yl}-phényl)-2-oxo-5-oxazol idi nyl-méthyl]acétam ide;
N-[(5S)-3-(3-fluoro-4-{3-[4-(2-méthoxyphényl)-pyrazol-1-yl]pyrrolidin-1-yl}-phényl)-2-oxo-5-oxazolidinyl-méthyl]acétamide;
N-((5S)-3-{3-fluoro-4-[3-(4-acétylpyrazol-1-yl)pyrrolidin-1-yl]-phényl}-2-oxo-5-oxazolidinyl-méthyl)acétamide;
N-((5S)-3-{3-fluoro-4-[3-(3-phénylpyrazol-1-yl)pyrrolidin-1-yl]-phényl}-2-oxo-5-oxazolidinyl-méthyl)acétamide;
N-[(5S)-3-(3-fluoro-4-{3-[4-(4-fluorophényl)-pyrazol-1-yl]pyrrolidin-1-yl}-phényl)-2-oxo-5-oxazolidinyl-méthyl]acétamide;
N-[(5S)-3-(3-fluoro-4-{3-[3-(2-fluorophényl)-pyrazol-1-yl]pyrrolidin-1-yl}-phényl)-2-oxo-5-oxazolidinyl-méthyl]acétamide;
N-((5S)-3-{3-fluoro-4-[3-(3-trifluorométhyl-pyrazol-1-yl)pyrrolidin-1-yl]-phényl}-2-oxo-5-oxazolidinyl-méthyl)acétamide;
N-[(5S)-3-(3-fluoro-4-{3-[3-{4-trifluorométhyl-phényl)-pyrazol-1-yl]pyrrolidin-1-yl}-phényl)-2-oxo-5-oxazol idi nyl-méthyl]acétam ide;
N-((5S)-3-{3-fluoro-4-[3-(4-pyridin-2-yl-[1,2,3]triazol-2-yl)pyrrolidin-1-yl]-phényl}-2-oxo-5-oxazolidinyl-méthyl)acétamide;
N-[(5S)-3-(3-fluoro-4-{3-[4-(3-cyanophényl)-[1,2,3]triazol-2-yl]pyrrolidin-1-yl}-phényl)-2-oxo-5-oxazolidinyl-méthyl]acétamide;
N-{{5S)-3-[3,5-difluoro-4-[3-([1,2,3]triazol-2-yl-pyrrolidin-1-yl)-phényl]-2-oxo-5-oxazolidinyl-méthyl}acétamide;
N-{(5S)-3-[3,5-difluoro-4-[3-([1,2,3]triazol-1-yl-pyrrolidin-1-yl)-phényl]-2-oxo-5-oxazolidinyl-méthyl}acétamide;
N-{(5S)-3-[3,5-difluoro-4-[3-(tetrazol-2-yl-pyrrolidin-1-yl)-phényl]-2-oxo-5-oxazolidinyl méthyl}acétamide;
N-{(5S)-3-[3,5-difluoro-4-[3-(tetrazol-1-yl-pyrrolidin-1-yl)-phényl]-2-oxo-5-oxazolidinyl-méthyl}acétamide;
N-((5S)-3-{3-fluoro-4-[3(R)-(2-triazolyl)pyrrolidin-1-yl]-phényl}-2-oxo-5-oxazolidinyl-méthyl)acétamide;
N-((5S)-3-{3-fluoro-4-[3(R)-(1-thazolyl)pyrrolidin-1-yl]-phényl}-2-oxo-5-oxazolidinyl-méthyl)acétamide;
N-((5S)-3-[3-fluoro-4-[3(R)-(1-imidazolyl)pyrrolidin-1-yl]-phényl}-2-oxo-5-oxazolidinyl-méthyl)acétamide;
N-((5S)-3-{3-fluoro-4-[3(R)-(2-tetrazolyl)pyrrolidinyl]-phényl}-2-oxo-5-oxazolidinyl-méthyl)acétamide;
N-((5S)-3-{3-fluoro-4-[3(R)-(1-tetrazolyl)pyrrolidinyl]-phényl}-2-oxo-5-oxazolidinyl-méthyl)acétamide;
N-{(5S)-3-[3-fluoro-4-(3(R)-[1,2,4]triazol-1-yl-pyrrolidin-1-yl)-phényl]-2-oxo-5-oxazolidinyl-méthyl}acétamide;
N-((5S)-3-{3-fluoro-4-[3(R)-(benzotriazolyl)pyrrolidin-1-yl]-phényl}-2-oxo-5-oxazolidinyl-méthyl)acétamide;
N-((5S)-3-{3-fluoro-4-[3(R)-(4-nitropyrazolyl)pyrrolidinyl]-phényle}-2-oxo-5-oxazolidinyl-méthyl)acétamide;
N-((5S)-3-{3-fluoro-4-[3(R)-(5-p-tolyltetrazol-2-yl)pyrrolidin-1-yl]-phényl}-2-oxo-5-oxazolidinyl-méthyl)acétamide;
N-((5S)-3-{3-fluoro-4-[3(R)-(4-pyrimidin-4-yl-pyrazol-1-yl)pyrrolidin-1-yl]-phényl}-2-oxo-5-oxazol idi nyl-méthyl)acétam ide;
N-((5S)-3-{3-fluoro-4-[3(R)-(p-pyrazin-2-yl-pyrazol-yl)pyrrolidin-1-yl]-phényl}-2-oxo-5-oxazolidinyl-méthyl)acétamide;
N-((5S)-3-{3-fluoro-4-[3(R)-(5-phényltetrazol-2-yl)pyrrolidin-1-yl]-phényl}-2-oxo-5-oxazolidinyl-méthyl)acétamide;
N-((5S)-3-(3-fluoro-4-[3(R)-(5-méthylsulfanyltetrazol-2-yl)pyrrolidin-1-yl]-phényl)-2-oxo-5-oxazolidinyl-méthyl)acétamide;
N-((5S)-3-{3-fluoro-4-[3(R)-(5-thiophen-2-yl-tetrazol-2-yl)pyrrolidin-1-yl]-phényl}-2-oxo-5-oxazolidinyl-méthyl)acétamide ;
N-((5S)-3-{3-fluoro-4-[3(R)-(5-méthyltetrazol-2-yl)pyrrolidin-1-yl]-phényl}-2-oxo-5-oxazolidinyl-méthyl)acétamide ;
N-((5S)-3-{3-fluoro-4-[3(R)-(4-bromopyrazol-1-yl)pyrrolidin-1-yl]-phényl}-2-oxo-5-oxazolidinyl-méthyl)acétamide ;
N-((5S)-3-{3-fluoro-4-[3(R)-(4-pyridin-4-yl-pyrazol-1-yl)pyrrolidin-1-yl]-phényl}-2-oxo-5-oxazolidinyl-méthyl)acétamide ;
N-[(5S)-3-(3-fluoro-4-{3(R)-[4-(4-nitrophényl)-pyrazol-1-yl]pyrrolidin-1-yl}-phényl)-2-oxo-5-oxazolidinyl-méthyl]acétamide ;
N-[(5S)-3-(3-fluoro-4-{3(R)-[4-(2-trifluorométhyl-phényl)-pyrazol-1-yl]pyrrolidin-1-yl}-phényl)-2-oxo-5-oxazolidinyl-méthyl]acétamide ;
N-[(5S)-3-(3-fluoro-4-{3(R)-[4-(2-méthoxyphényl)-pyrazol-1-yl]pyrrolidin-1-yl}-phényl)-2-oxo-5-oxazolidinyl-méthyl]acétamide ;
N-((5S)-3-{3-fluoro-4-[3(R)-{4-acétylpyrazol-1-yl)pyrrolidin-1-yl]-phényl}-2-oxo-5-oxazolidinyl-méthyl)acétamide ;
N-((5S)-3-{3-fluoro-4-[3(R)-(3-phénylpyrazol-1-yl)pyrrolidin-1-yl]-phényl}-2-oxo-5-oxazolidinyl-méthyl)acétamide ;
N-[(55)-3-(3-fluoro-4-{3(R)-[4-(4-fluorophényl)-pyrazol-1-yl]pyrrolidin-1-yl}-phényl)-2-oxo-5-oxazolidinyl-méthyl]acétamide ;
N-[(5S)-3-(3-fluoro-4-{3(R)-[3-{2-fluorophényl)-pyrazol-1-yl]pyrrolidin-1-yl}-phényl)-2-oxo-5-oxazolidinyl-méthyl]acétamide ;
N-((5S)-3-{3-fluoro-4-[3(R)-(3-trifluorométhyl-pyrazol-1-yl)pyrrolidin-1-yl]-phényl}-2-oxo-5-oxazolidinyl-méthyl)acétamide ;
N-[(5S)-3-(3-fluoro-4-{3(R)-[3-(4-trifluorométhyl-phényl)-pyrazol-1-yl]pyrrolidin-1-yl}-phényl)-2-oxo-5-oxazolidinyl-méthyl]acétamide ;
N-((5S)-3-{3-fluoro-4-[3(R)-(4-pyridin-2-yl-[1,2,3]tria20l-2-yl)pyrrolidin-1-yl]-phényl}-2-oxo-5-oxazolidinyl-méthyl)acétamide ;
N-[(55)-3-(3-fluoro-4-(3(R)-[4-(3-cyanophényl)-[1,2,3]triazol-2-yl]pyrrolidin-1-yl}-phényl)-2-oxo-5-oxazolidinyl-méthyl]acétamide ;
N-{(5S)-3-[3,5-difluoro-4-[3(R)-([1,2,3]triazol-2-yl-pyrrolidin-1-yl)-phényl]-2-oxo-5-oxazolidinyl-méthyl}acétamide ;
N(5S)-3-[3,5-difluoro-4-[3(R)-([1,2,3]triazol-1-yl-pyrrolidin-1-yl)-phényl]-2-oxo-5-oxazolidinyl-méthyl}acétamide ;
N-((5S)-3-[3,5-difluoro-4-[3(R)-(tetrazol-2-yl-pyrrolidin-1-yl)-phényl]-2-oxo-5-oxazolidinyl-méthyl}acétamide ;
N-{(5S)-3-[3,5-difluoro-4-[3(R)-(tetrazol-1-yl-pyrrolidin-1-yl)-phényl]-2-oxo-5-oxazolidinyl-méthyl}acétamide ;
N-((5S)-3-{3-fluoro-4-[3(S)-(2-triazolyl)pyrrolidin-1-yl]-phényl}-2-oxo-5-oxazolidinyl-méthyl)acétamide ;
N-((5S)-3-{3-fluoro-4-[3(S)-(1-triazolyl)pyrrolidin-1-yl]-phényl}-2-oxo-5-oxazolidinyl-méthyl)acétamide ;
N-((5S)-3-[3-fluoro-4-[3(S)-(1-imidazolyl)pyrrolidin-1-yl]-phényl}-2-oxo-5-oxazolidinyl-méthyl)acétamide ;
N-((5S)-3-{3-fluoro-4-[3(S)-(2-tetrazolyl)pyrrolidinyl]-phényl}-2-oxo-5-oxazolidinyl-méthyl)acétamide ;
N-((5S)-3-{3-fluoro-4-[3(S)-(1-tetrazolyl)pyrrolidinyl]-phényl}-2-oxo-5-oxazolidinyl-méthyl)acétamide ;
N-{(5S)-3-[3-fluoro-4-(3(S)-[1,2,4]triazol-1-yl-pyrrolidin-1-yl)-phényl]-2-oxo-5-oxazolidinyl-méthyl}acétamide ;
N-((5S)-3-(3-fluoro-4-[3(S)-(benzotriazolyl)pyrrolidin-1-yl]-phényl)-2-oxo-5-oxazolidinyl-méthyl)acétamide ;
N-((5S)-3-{3-fluoro-4-[3(S)-(4-nitropyrazolyl)pyrrolidinyl]-phényl}-2-oxo-5-oxazolidinyl-méthyl)acétamide ;
N-((5S)-3-{3-fluoro-4-[3(S)-(5-p-tolyltetrazol-2-yl)pyrrolidin-1-yl]-phényl}-2-oxo-5-oxazolidinyl-méthyl)acétamide ;
N-((5S)-3-{3-fluoro-4-[3(S)-(4-pyrimidin-4-yl-pyrazol-1-yl)pyrrolidin-1-yl]-phényl}-2-oxo-5-oxazolidinyl-méthyl)acétamide ;
N-((5S)-3-{3-fluoro-4-[3(S)-(4-pyrazin-2-yl-pyrazol-1-yl)pyrrolidin-1-yl]-phényl}-2-oxo-5-oxazolidinyl-méthyl)acétamide ;
N-((5S)-3-{3-fluoro-4-[3(S)-(5-phényltetrazol-2-yl)pyrrolidin-1-yl]-phényl}-2-oxo-5-oxazolidinyl-méthyl)acétamide ;
N-((5S)-3-{3-fluoro-4-[3(S)-(5-méthylsulfanyltetrazol-2-yl)pyrrolidin-1-yl]-phényl}-2-oxo-5-oxazolidinyl-méthyl)acétamide ;
N-((5S)-3-{3-fluoro-4-[3(S)-(5-thiophen-2-yl-tetrazol-2-yl)pyrrolidin-1-yl]-phényl}-2-oxo-5-oxazol idi nyl-méthyl)acétam ide;
N-((5S)-3-{3-fluoro-4-[3(S)-(5-méthyltetrazol-2-yl)pyrrolidin-1-yl]-phényl}-2-oxo-5-oxazolidinyl-méthyl)acétamide ;
N-((5S)-3-{3-fluoro-4-[3(S)-(4-bromopyrazol-1-yl)pyrrolidin-1-yl]-phényl}-2-oxo-5-oxazolidinyl-méthyl)acétamide ;
N-((5S)-3-{3-fluoro-4-[3(S)-(4-pyridin-4-yl-pyrazol-1-yl)pyrrolidin-1-yl]-phényl}-2-oxo-5-oxazolidinyl-méthyl)acétamide ;
N-[(5S)-3-(3-fluoro-4-{3(S)-[4-(4-nitrophényl)-pyrazol-1-yl]pyrrolidin-1-yl}-phényl)-2-oxo-5-oxazolidinyl-méthyl]acétamide ;
N-[(5S)-3-(3-fluoro-4-{3(S)-[4-(2-trifluorométhyl-phényl)-pyrazol-1-yl]pyrrolidin-1-yl}-phényl)-2-oxo-5-oxazolidinyl-méthyl]acétamide ;
N-[(5S)-3-(3-fluoro-4-{3(S)-[4-(2-methoxyphényl)-pyrazol-1-yl]pyrrolidin-1-yl}-phényl)-2-oxo-5-oxazolidinyl-méthyl]acétamide ;
N-((5S)-3-{3-fluoro-4-[3(S)-(4-acetylpyrazol-1-yl)pyrrolidin-1-yl]-phényl}-2-oxo-5-oxazolidinyl-méthyl)acétamide ;
N-((5S)-3-{3-fluoro-4-[3(S)-(3-phénylpyrazol-1-yl)pyrrolidin-1-yl]-phényl}-2-oxo-5-oxazolidinyl-méthyl)acétamide ;
N-[(5S)-3-(3-fluoro-4-{3(S)-[4-(4-fluorophényl)-pyrazol-1-yl]pyrrolidin-1-yl}-phényl)-2-oxo-5-oxazolidinyl-méthyl]acétamide ;
N-[(5S)-3-(3-fluoro-4-{3(S)-[3-(2-fluorophényl)-pyrazol-1-yl]pyrrolidin-1-yl}-phényl)-2-oxo-5-oxazolidinyl-méthyl]acétamide ;
N-((5S)-3-{3-fluoro-4-[3(S)-(3-trifluorométhyl-pyrazol-1-yl)pyrrolidin-1-yl]-phényl}-2-oxo-5-oxazolidinyl-méthyl)acétamide ;
N-[(5S)-3-(3-fluoro-4-{3(S)-[3-(4-trifluorométhyl-phényl)-pyrazol-1-yl]pyrrolidin-1-yl}-phényl)-2-oxo-5-oxazolidinyl-méthyl]acétamide ;
N-((5S)-3-{3-fluoro-4-[3(S)-(4-pyridin-2-yl-[1,2,3]triazol-2-yl)pyrrolidin-1-yl]-phényl}-2-oxo-5-oxazolidinyl-méthyl)acétamide ;
N-[(5S)-3-(3-fluoro-4-(3(S)-[4-(3-cyanophényl)-[1,2,3]triazol-2-yl]pyrrolidin-1-yl)-phényl)-2-oxo-5-oxazolidinyl-méthyl]acétamide ;
N-{(5S)-3-[3,5-fifluoro-4-[3(S)-([1,2,3]triazol-2-yl-pyrrolidin-1-yl)phényl]-2-oxo-5-oxazolidinyl-méthyl}acétamide ;
N-{(5S)-3-[3,5-difluoro-4-[3(S)-([1,2,3]triazol-1-yl-pyrrolidin-1-yl)phényl]-2-oxo-5-oxazolidinyl-méthyl}acétamide ;
N-{(5S)-3-[3,5-difluoro-4-[3(S)-(tetrazol-2-yl-pyrrolidin-1-yl)phényl]-2-oxo-5-oxazolidinyl-méthyl}acétamide; et
N-{(5S)-3-[3,5-difluoro-4-[3(S)-(tetrazol-1-yl-pyrrolidin-1-yl)phényl]-2-oxo-5-oxazolidinyl-méthyl}acétamide.

7. Procédé pour préparer un composé de formule (I) sous forme libre ou de sel, solvate, hydrate ou énantiomère acceptable sur le plan pharmaceutique, qui comprend:
(i) la réaction d'un intermédiaire de formule (II), 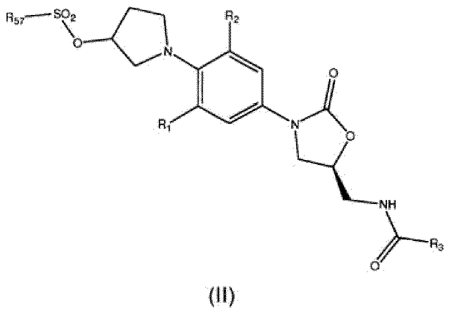 dans laquelle R₁, R₂ et R₃ sont tels que définis précédemment, et R₅₇ est choisi parmi le méthyle, phényle, p-tolyle, p-bromophényle, p-nitrophényle, trifluorométhyle et le 2,2,2-trifluoroéthyle, avec un intermédiaire de formule RH (III), dans laquelle R est tel que défini précédemment ; ou
b) la réaction d'un intermédiaire de formule (IV), 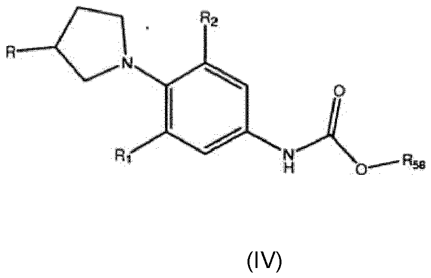 dans laquelle R, R₁, et R₂ sont tels que définis précédemment et R₅₈ est choisi parmi un groupe alkyle en C1 à C6 linéaire ou ramifié, et benzyle éventuellement substitué sur le noyau phényle par jusqu'à trois groupes alkyle en C1 à C6 linéaires ou ramifiés, avec un intermédiaire de formule (V), 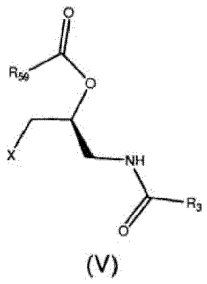 dans laquelle R₃ est tel que défini précédemment, R₅₉ est un groupe alkyle en C1 à C6 linéaire ou ramifié, et X est un atome d'halogène ; et
(ii) la récupération du composé résultant de formule (I) sous forme libre ou de sel, solvate, hydrate ou énantiomère acceptable sur le plan pharmaceutique.

8. Procédé selon la revendication 7 dans lequel R₅₇ est méthyl, R₅₈ est benzyle, R₅₉ est méthyle, et X est bromure.

9. Composition pharmaceutique comprenant une quantité efficace sur le plan thérapeutique du composé de formule (I) tel que défini dans l'une quelconque des revendications 1 à 6, conjointement avec la quantité appropriée d'excipients ou de vecteurs pharmaceutiques.

10. Composé de formule (I) tel que défini dans l'une quelconque des revendications 1 à 6 pour une utilisation en tant que médicament.

11. Composé de formule (I) tel que défini dans l'une quelconque des revendications 1 à 6 pour une utilisation dans le traitement d'infections bactériennes.
